(19) Europäisches Patentamt
European Patent Office
Office européen des brevets

(11) Veröffentlichungsnummer: **0 072 346**
**B1**

(12) # EUROPÄISCHE PATENTSCHRIFT

(45) Veröffentlichungstag der Patentschrift: **09.04.86**

(51) Int. Cl.⁴: **C 07 D 487/04**, A 61 K 31/505 // (C07D487/04, 239:00, 209:00)

(21) Anmeldenummer: **82810321.8**

(22) Anmeldetag: **28.07.82**

(54) **Weitere neue Amidine, Verfahren zu ihrer Herstellung, diese enthaltende pharmazeutische Präparate und ihre Verwendung.**

(30) Priorität: **03.08.81 CH 5004/81**

(43) Veröffentlichungstag der Anmeldung: **16.02.83 Patentblatt 83/7**

(45) Bekanntmachung des Hinweises auf die Patenterteilung: **09.04.86 Patentblatt 86/15**

(84) Benannte Vertragsstaaten: **AT BE CH DE FR IT LI LU NL SE**

(56) Entgegenhaltungen:
**EP - A - 0 008 950**
**EP - A - 0 035 474**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Klybeckstrasse 141, CH-4002 Basel (CH)**

(72) Erfinder: **Renner, Ulrich, Dr., Auf der Bischoffhöhe 26, CH-4125 Riehen (CH)**
Erfinder: **Jaeggi, Knut A., Dr., General Guisan-Strasse 44, CH-4054 Basel (CH)**

**EP 0 072 346 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft neue Amidine, insbesondere N,N'-überbrückte Carbonsäureamidine der allgemeinen Formel

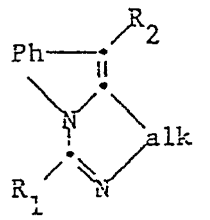

(I),

worin $R_1$ gegebenenfalls in p-Stellung durch Niederalkylthio mit bis und mit 4 C-Atomen durch Niederalkansulfinyl mit bis und mit 4 C-Atomen, Niederalkoxy mit bis und mit 4 C-Atomen oder Halogen der Atomnummer bis und mit 35 substituiertes Phenyl, Thienyl oder gegebenenfalls durch Niederalkyl mit bis und mit 4 C-Atomensubstituiertes Pyridyl bedeutet, $R_2$ Carboxymethyl oder in zweiter Linie Niederalkoxycarbonylmethyl mit bis und mit 5 C-Atomen ist, Ph gegebenenfalls in p-Stellung zum Stickstoffatom durch Niederalkoxy mit bis und mit 4 C-Atomen oder Halogen der Atomnummer bis und mit 35 substituiertes 1,2-Phenylen darstellt und alk Vinylen bedeutet,und ihre Salze, mit der Massgabe, dass, wenn $R_1$ p-Methylthiophenyl ist und Ph in p-Stellung zum N-Atom durch Fluor substituiertes 1,2-Phenylen bedeutet, $R_2$ von Carboxymethyl oder Ethoxycarbonylmethyl verschieden ist, und der weiteren Massgabe, dass, wenn $R_1$ Phenyl ist und Ph unsubstituiertes 1,2-Phenylen bedeutet oder wenn $R_1$ p-Chlorphenyl ist und Ph in p-Stellung zum N-Atom durch Methoxy substituiertes 1,2-Phenylen darstellt, $R_2$ jeweils von Ethoxycarbonylmethyl verschieden ist, Verfahren zu ihrer Herstellung, diese enthaltende pharmazeutische Präparate und ihre Verwendung als Arzneimittelstoffe.

In der vorliegenden Beschreibung sind unter "niederen" organischen Resten und Verbindungen vorzugsweise solche zu verstehen, die bis und mit 7, vor allem bis und mit 4 Kohlenstoffatome enthalten.

Die vor- und nachstehend verwendeten Allgemeindefinitionen haben im Rahmen der vorliegenden Beschreibung in erster Linie die folgenden Bedeutungen.

Niederalkylthio ist z.B. Methyl-, Ethyl-, n-Propyl-, Isopropyl-, n-Butyl-, Isobutyl-, sek.-Butyl-, tert.-Butyl-,

n-Pentyl-, n-Hexyl- oder n-Heptylthio, Niederalkansulfinyl ist z.B. Methan-, Ethan- oder n-Propansulfinyl.

Niederalkoxy ist z.B. Methoxy, Ethoxy, n-Propyloxy, Isopropyloxy, n-Butyloxy, Isobutyloxy, sek.-Butyloxy oder tert.-Butyloxy.

Halogen ist z.B. Halogen bis und mit Atomnummer 35, wie Fluor, Chlor oder Brom, ferner Jod.

Niederalkyl ist z.B. Methyl, Ethyl, n-Propyl, Isopropyl, n-Butyl, Isobutyl, sek.-Butyl, tert.-Butyl, ferner ein Pentyl-, Hexyl- oder Heptylrest.

Thienyl ist z.B. 2- oder 3-Thienyl und Pyridyl 2-, 3- oder 4-Pyridyl.

Niederalkoxycarbonyl ist z.B. Methoxy-, Ethoxy-, n-Propyloxycarbonyl, ferner ein Butyloxy-, Pentyloxy-, Hexyloxy- oder Heptyloxycarbonylrest.

Salze von erfindungsgemässen Verbindungen der Formel I sind vorzugsweise pharmazeutisch verwendbare Salze, wie entsprechende Säureadditionssalze und/oder, wenn $R_2$ 1-Carboxymethyl ist, innere Salze oder Salze mit Basen. Geeignete Säureadditionssalze sind beispielsweise Salze mit anorganischen Säuren, wie Mineralsäuren, oder organischen Säuren, wie Sulfaminsäuren, z.B. Cyclohexylsulfaminsäure, gegebenenfalls ungesättigten Dicarbonsäuren oder gegebenenfalls durch Hydroxy zusätzlich substituierten bzw. zusätzlich Oxo und/oder Carboxy aufweisenden Carbonsäuren, oder Sulfonsäuren. Mineralsäuren sind beispielsweise Schwefelsäure oder Halogenwasserstoffsäuren, wie Brom- oder Chlorwasserstoffsäure. Als gegebenenfalls ungesättigte Dicarbonsäuren kommen z.B. Oxalsäure, Malon-, Fumar- oder Maleinsäure in Betracht, als gegebenenfalls durch Hydroxy zusätzlich substituierte bzw. zusätzlich Oxo und/oder Carboxy aufweisende Carbonsäuren werden z.B. Wein-, Aepfel-, Brenztrauben- oder Citronensäure verwendet. Sulfonsäuren sind z.B. Benzol-, p-Toluol- oder Methansulfonsäure.

Geeignete Salze mit Basen sind beispielsweise Metall-, wie Alkali- oder Erdalkalimetallsalze, z.B. Natrium-, Kalium-, Magnesium- oder Calciumsalze, Uebergangsmetallsalze, wie Zink- oder Kupfersalze, oder Salze mit Ammoniak oder von substituierten organischen Aminen, wie Morpholin, Thiomorpholin, Piperidin, Pyrrolidin, wie Mono-, Di- bzw. Triniederalkylaminen oder Mono-, Di- bzw. Trihydroxyniederalkylaminen, z.B. Mono-, Di- oder Triethanolamin sowie Tris-(hydroxymethyl)-methylamin oder Diisopropanolamin. Mononiederalkylamine sind z.B. Diethyl- oder Dipropylamin, und als Triniederalkylamine kommen z.B. Triethyl-, Tributylamin oder Dimethylpropylamin in Betracht. Weiterhin können Salze mit basischen Aminsäuren, wie Lysin, Arginin, Histidin oder Ornithin, oder mit Aminen gebildet werden, die sich von Monosacchariden ableiten, wie N-Methyl-D-glucamin.

Die Verbindungen der Formel I weisen wertvolle pharmakologische Eigenschaften auf. Insbesondere besitzen sie eine ausgeprägte antinociceptive (analgetische) Wirkung, die sich beispielsweise anhand des Essigsäure-Writhing-Syndroms an der Ratte im Dosisbereich von etwa 1 bis etwa 30 mg/kg p.o. und im Phenyl-p-Benzochinon-Writhing-Test an der Maus im Dosisbereich von etwa 1 bis etwa 30 mg/kg p.o. zeigen lässt.

Ferner weisen sie eine deutliche antiinflammatorische und antiarthritische Wirkung auf, die sich durch Hemmung des Kaolinpfotenoedems bei der Normalratte im Dosisbereich von etwa 10 bis 100 mg/kg p.o. nachweisen lässt und die sich zudem in der Hemmung des Carrageenin-Pfotenoedems an der Ratte analog der von Pasquale et al., Agents and Actions, 5, 256 (1976), beschriebenen Methode in Dosen von etwa 3 bis etwa 300 mg/kg p.o. zeigen lässt.

Ausserdem hemmen Verbindungen der Formel I in kurativer Applikation bei viermaliger Gabe von etwa 10 bis 100 mg/kg p.o. das Kaolinpfotenoedem der Adjuvans-Arthritis-Ratte.

Die Verbindungen der Formel I sind deshalb vorzüglich geeignet als Arzneimittel zur Behandlung entzündlicher Erkrankungen, vor allem solcher des rheumatischen bzw. arthritischen Bereichs, als Antiphlogistika und/oder als periphere Analgetika.

In der EP-Patentanmeldung Nr. 35 474 werden ebenfalls analgetisch und antiinflammatorisch wirksame Pyrimidoindol-Verbindungen beschrieben. Demgegenüber weisen die in Beispiel II genannten beiden letzten Verbindungen ein andersartiges Aktivitätsverhalten auf.

Die Erfindung betrifft beispielsweise Verbindungen der Formel I, worin $R_1$ gegebenenfalls in p-Stellung durch Niederalkylthio mit bis und mit 4 C-Atomen, wie Methylthio, durch Niederalkansulfinyl mit bis und mit 4 C-Atomen, wie Methansulfinyl, Niederalkoxy mit bis und mit 4 C-Atomen, wie Methoxy, oder Halogen der Atomnummer bis und mit 35, wie Fluor, substituiertes Phenyl, Thienyl, wie 2-Thienyl, oder gegebenenfalls durch Niederalkyl mit bis und mit 4 C-Atomen, wie Methyl, substituiertes Pyridyl, wie 2-Picolinyl, bedeutet, $R_2$ Carboxymethyl oder in zweiter Linie Niederalkoxycarbonylmethyl mit bis und mit 5 C-Atomen, wie Ethoxycarbonylmethyl, ist, Ph gegebenenfalls in p-Stellung zum Stickstoffatom durch Niederalkoxy mit bis und mit 4 C-Atomen, wie Methoxy, oder Halogen der Atomnummer bis und mit 35, wie Fluor, substituiertes 1,2-Phenylen darstellt und alk Vinylen bedeutet, mit der Massgabe, dass, wenn $R_1$ p-Methylthiophenyl ist und Ph in p-Stellung zum N-Atom durch Fluor substituiertes 1,2-Phenylen bedeutet, $R_2$ von Carboxymethyl oder Ethoxycarbonylmethyl verschieden ist, und der weiteren Massgabe, dass, wenn $R_1$ Phenyl ist und Ph unsubstituiertes 1,2-Phenylen bedeutet oder wenn $R_1$ p-Chlorphenyl ist und Ph in p-Stellung zum N-Atom durch Methoxy substituiertes 1,2-Phenylen darstellt, $R_2$ jeweils von Ethoxycarbonylmethyl verschieden ist.

Die Erfindung betrifft bevorzugt Verbindungen der Formel I, worin $R_1$ unsubstituiertes Phenyl, p-Methoxyphenyl oder p-Methansulfinyl bedeutet, $R_2$ jeweils Ethoxycarbonylmethyl ist, Ph jeweils in p-Stellung zum N-Atom durch Fluor substituiertes 1,2-Phenylen darstellt und alk jeweils Vinylen ist, worin $R_1$ unsubstituiertes 2-Thienyl, unsubstituiertes Phenyl, p-Methansulfinylphenyl oder p-Methoxyphenyl bedeutet, $R_2$ jeweils Carboxymethyl ist, Ph jeweils in p-Stellung zum N-Atom durch Fluor substituiertes 1,2-Phenylen darstellt und alk jeweils Vinylen ist, worin $R_1$ jeweils unsubstituiertes 2-Thienyl bedeutet, $R_2$ Carboxymethyl oder Ethoxycarbonylmethyl ist, Ph jeweils unsubstituiertes 1,2-Phenylen darstellt und alk jeweils Vinylen ist, worin $R_1$ unsubstituiertes Phenyl bedeutet, $R_2$ Carboxymethyl ist, Ph unsubstituiertes 1,2-Phenylen darstellt und alk Vinylen ist, oder worin $R_1$ 2-Picolinyl oder p-Fluorphenyl bedeutet, $R_2$ jeweils Carboxymethyl oder Ethoxycarbonylmethyl ist, Ph jeweils in p-Stellung zum N-Atom durch Methoxy substituiertes 1,2-Phenylen darstellt und alk jeweils Vinylen ist, und ihre Salze.

Die Erfindung betrifft bevorzugt Verbindungen der Formel I, worin $R_1$ unsubstituiertes Phenyl, p-Methoxyphenyl oder p-Methansulfinyl bedeutet, $R_2$ jeweils Ethoxycarbonylmethyl ist, Ph jeweils in p-Stellung zum N-Atom durch Fluor substituiertes 1,2-Phenylen darstellt und alk jeweils Vinylen ist, worin $R_1$ unsubstituiertes Phenyl, p-Methansulfinylphenyl oder p-Methoxyphenyl bedeutet, $R_2$ jeweils Carboxymethyl ist, Ph jeweils in p-Stellung zum N-Atom durch Fluor substituiertes 1,2-Phenylen darstellt und alk jeweils Vinylen ist, worin $R_1$ jeweils unsubstituiertes Thienyl bedeutet, $R_2$ Carboxymethyl oder Ethoxycarbonylmethyl ist, Ph jeweils unsubstituiertes 1,2-Phenylen darstellt und alk jeweils Vinylen ist, worin $R_1$ unsubstituiertes Phenyl bedeutet, $R_2$ Carboxymethyl ist, Ph unsubstituiertes 1,2-Phenylen darstellt und alk Vinylen ist, oder worin $R_1$ 2-Picolinyl oder p-Fluorphenyl bedeutet, $R_2$ jeweils Carboxymethyl oder Ethoxycarbonylmethyl ist, Ph jeweils in p-Stellung zum N-Atom durch Methoxy substituiertes 1,2-Phenylen darstellt und alk jeweils Vinylen ist, und ihre Salze.

Die Erfindung betrifft in allererster Linie Verbindungen der Formel I, worin Ph unsubstituiertes 1,2-Phenylen bedeutet und $R_1$ Phenyl und $R_2$ Carboxymethyl oder $R_1$ 2-Thienyl und $R_2$ Carboxy- oder Ethoxycarbonylmethyl darstellt, oder Ph in p-Stellung zum N-Atom durch Fluor substituiertes 1,2-Phenylen bedeutet und $R_1$ Phenyl, p-Methansulfinylphenyl oder p-Methoxyphenyl und $R_2$ Carboxy- oder Ethoxycarbonylmethyl darstellt, oder Ph in p-Stellung zum N-Atom durch Methoxy substituiertes 1,2-Phenylen bedeutet und $R_1$ p-Fluorphenyl oder 2-Picolinyl und $R_2$ Carboxy- oder Ethoxycarbonylmethyl darstellt, wobei alk jeweils Vinylen bedeutet, und ihre Salze.

Die Erfindung betrifft namentlich Verfahren zur Herstellung der in den Beispielen genannten Verbindungen der Formel I, und ihre Salze, insbesondere ihrer pharmazeutisch verwendbaren Salze.

Die Erfindung betrifft namentlich die in den Beispielen genannten Verbindungen der Formel I und ihre Salze, insbesondere ihre pharmazeutisch verwendbaren Salze.

Die Verbindungen der Formel I und ihre Salze können nach an sich bekannten Methoden hergestellt werden, beispielsweise indem man aus Verbindungen der allgemeinen Formel

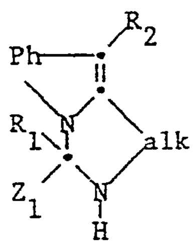

(II),

worin $Z_1$ gegebenenfalls funktionell abgewandeltes Hydroxy oder die Mercaptogruppe bedeutet, oder Salzen davon unter Einführung einer zusätzlichen Bindung $H-Z_1$ abspaltet und gewünschtenfalls eine erfindungsgemäss erhältliche Verbindung in eine andere Verbindung der Formel I bzw. eine erfindungsgemäss erhältliche freie Verbindung in ein Salz oder ein verfahrensgemäss erhältliches Salz in die freie Verbindung bzw. in ein anderes Salz überführt.

Funktionell abgewandeltes Hydroxy ist beispielsweise durch ein Niederalkanol, wie Methanol oder Ethanol, oder einen gegebenenfalls substituierten aromatischen Alkohol, wie Phenol, verethertes Hydroxy oder mit einer anorganischen Säure, wie einer Mineralsäure, z.B. Halogenwasserstoffsäure, wie Chlorwasserstoffsäure, verestertes Hydroxy und bedeutet beispielsweise Niederalkoxy, wie Methoxy oder gegebenenfalls substituiertes Aryloxy, wie Phenoxy, oder Halogen, wie Chlor oder Brom.

Die Abspaltung von $H-Z_1$ erfolgt in üblicher Weise, beispielsweise spontan, thermisch, d.h. unter Erwärmen, und/oder in Gegenwart eines katalytischen Mittels. Die thermische Abspaltung verläuft üblicherweise in einem Temperaturbereich von etwa 50° bis etwa 200°c. Als katalytische Mittel verwendet man z.B. basische bzw. saure Katalysatoren, wobei als Basen beispielsweise Alkalimetallhydroxide, -amide bzw. -hydride, wie Kaliumhydroxid, Natriumamid bzw. Natriumhydrid, Metalloxide, wie Aluminiumoxid, vor allem organische Stickstoffbasen, wie tertiäre Amine, z.B. Pyridin, Chinolin oder N,N-Dimethylanilin, und als saure Katalysatoren beispielsweise Mineralsäuren oder saure Salze bzw. Anhydride davon, wie Schwefelsäure oder Phosphorsäuren, Hydrogensulfate, wie Alkalimetallhydrogensulfate, z.B. Kaliumhydrogensulfat, Phosphorpentoxid, oder Mineralsäurehalogenide, wie Phosphor(III)-, Phosphor(V)chlorid oder Phophoroxychlorid, Schwefelsäurehalogenide, z.B. Sulfurylchlorid, eingesetzt werden. Dabei arbeitet man erforderlichenfalls in Gegenwart eines inerten Lösungs- bzw. Verdünnungsmittels, in einem geschlossenen Gefäss und/oder unter Inertgas z.B. Stickstoff.

Inerte Lösungs- bzw. Verdünnungsmittel sind gegebenenfalls substituierte Kohlenwasserstoffe, wie gegebenenfalls halogenierte aliphatische oder aromatische Kohlenwasserstoffe, z.B. Chloroform oder Chlorbenzol, Ether, wie aliphatische, cycloaliphatische oder aromatische Ether, z.B. Diethylether, Dioxan, Tetrahydrofuran, Diphenylether oder Anisol, Ketone, wie aliphatische Ketone, z.B. Aceton oder Methylethylketon, Amide, wie Dialkylamide, z.B. Dimethylformamid, oder Sulfoxide, wie Diniederalkylsulfoxide, z.B. Dimethylsulfoxid.

Ausgangsstoffe der Formel II können nach an sich bekannten Verfahren hergestellt werden, vorzugsweise in situ. So kann man beispielsweise Verbindungen der Formel

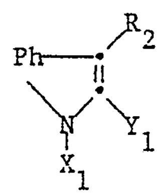

(IVa)

oder Salze davon, worin $X_1$ Wasserstoff und $Y_1$ eine Gruppe der Formel $-alk-NH-C(=Z'_1)(R_1)$ bzw. $X_1-C(=Z'_1)(R_1)$ und $Y_1$ eine Gruppe der Formel $-alk-NH_2$ und $Z'_1$ gegebenenfalls funktionell abgewandeltes Oxo ist, cyclisiert und gewünschtenfalls eine so erhältliche freie Verbindung der Formel II in eine andere freie Verbindung bzw. in ein Salz oder ein verfahrensgemäss erhältliches Salz in die freie Verbindung bzw. in ein anderes Salz überführt: So kann beispielsweise freies Hydroxy in üblicher Weise in Halogen oder mit einer Niederalkancarbonsäure verestertes Hydroxy überführt werden.

Funktionell abgewandeltes Oxo ist beispielsweise Thioxo, ketalisiertes oder thioketalisiertes Oxo, verestertes Dioxy oder Imino. Ketalisierte Oxoverbindungen sind beispielsweise Ketale mit Niederalkanolen, wie Methanol oder Ethanol, oder Niederalkandiolen, wie Ethylenglykol oder Propylenglykole, z.B. mit 1,3-Dihydroxypropan, und Thioketale sind beispielsweise Thioketale mit Niederalkanthiolen, z.B. Methanthiol oder Ethanthiol, oder Niederalkandithiolen, wie 1,2-Ethanthiol oder Propandithiolen, z.B. mit Propan-1,3-dithiol. Imino ist beispielsweise gegebenenfalls durch Niederalkyl oder Phenyl substituiertes Imino, wie N-Niederalkyl-, z.B.

N-Propylimino.

Die Cyclisierung erfolgt in bekannter Weise, beispielsweise in Gegenwart von Katalysatoren, wie sauren Katalysatoren. Solche sind

beispielsweise Mineralsäuren, wie Schwefelsäure oder Polyphosphorsäure, Mineralsäurehalogenide, wie Sulfurylchlorid oder Phosphorhalogenide, z.B. Phosphorpentachlorid, oder organische Sulfonsäuren, wie Benzol-, p-Toluol- oder Methansulfonsäure. Dabei arbeitet man erforderlichenfalls in einem der oben erwähnten inerten Lösungs- bzw. Verdünnungsmittel, vorzugsweise unter Erwärmen, z.B. im Temperaturbereich von etwa 20° bis etwa 200° C, in einem geschlossenen Gefäss und/oder unter Inertgas, z.B. Stickstoff.

In einer vorteilhaften Ausführungsform des vorstehend beschriebenen Verfahrens geht man von Verbindungen der Formel IV aus und führt die Cyclisierung zu Verbindungen der Formel II und die Abspaltung von H-$Z_1$ aus den Verbindungen der Formel II ohne Isolierung der Zwischenprodukte in situ durch.

Eine besonders vorteilhafte Ausführungsform des vorstehenden, über Verbindungen der Formel II verlaufenden Verfahrens besteht beispielsweise darin, dass man Verbindungen der Formel

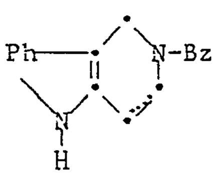 (IVd),

worin Bz ein gegebenenfalls substituierter α-Phenylniederalkylrest, vozugsweise Benzyl, bedeutet, insbesondere mit Benzylbromid quaternisiert, mit Hilfe von Cyaniden, wie Alkalimetallcyaniden, z.B. Natriumcyanid, die Bindung am quartären Stickstoff spaltet und in einer erhaltenen Verbindung der Formel IVe

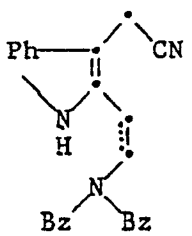 (IVe)

die Cyanogruppe wie gewünscht solvolysiert, die Benzylgruppen hydrogenolytisch in Gegenwart eines Hydrierungskatalysators, z.B. von Palladium, abspaltet und die nunmehr freie Aninoverbindung mit einer Verbindung der Formel

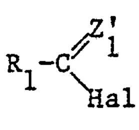,

worin $Z'_1$ gegebenenfalls funktionell abgewandeltes Oxo und Hal Halogen bedeutet, umsetzt und schliesslich mittels eines Cyclisierungsmittels, vorzugsweise eines Mineralsäurehalogenids, wie Phosphoroxychlorid oder Phosphorchlorid, d.h. Phosphor(III)- oder Phosphor(V)chlorid, zu einer Verbindung der Formel II umsetzt, aus der unter den Reaktionsbedingungen unter Kondensation direkt die entsprechende Verbindung der Formel I gebildet wird. Insbesondere wird diese Reaktion so durchgeführt, dass entsprechende 3,4-Dihydropyrimido[1,6-a] indol-essigsäuren bzw. -niederalkylester entstehen. Entsprechend müssen diese Dihydro-Derivate in der unten angeführten Weise zu den gewünschten Verbindungen der Formel I dehydriert werden. Die Verbindungen der Formel I bzw. Salze davon lassen sich ferner herstellen, indem man Verbindungen der Formel

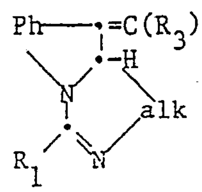 (V),

worin $R'_2$ gegebenenfalls wie unter $R_2$ angegeben verestertes Carboxy und $R_3$ Wasserstoff bedeutet, oder Salze davon isomerisiert und gewünschtenfalls eine so erhältliche freie Verbindung der Formel I in eine andere freie Verbindung bzw. in ein Salz davon oder ein verfahrensgemäss erhältliches Salz in die freie Verbindung bzw. in ein anderes Salz überführt.

Die Isomerisierung von Verbindungen der Formel V zu Verbindungen der Formel I erfolgt in üblicher Weise, erforderlichenfalls mit Hilfe von Säuren, wie Mineralsäuren, z.B. Schwefelsäure, von Basen, wie von Alkalimetallhydroxiden bzw. -carbonaten, z.B. Natriumhydroxid bzw. Kaliumcarbonat, oder wie von organischen Aminen, z.B. tertiären Aminen, wie Pyridin, oder durch Zufuhr von Energie, wie bei Temperaturen über 100° C, gegebenenfalls in Gegenwart eines katalytischen Mittels, wie eines Borates oder Phosphates, z.B. eines Alkalimetallborates oder -phosphates, und, wenn erforderlich, in einem Lösungs- bzw. Verdünnungsmittel, in einem geschlossenen Gefäss und/oder unter Inertgas, z.B. Stickstoff.

Inerte Lösungs- bzw. Verdünnungsmittel sind gegebenenfalls substituierte Kohlenwasserstoffe, wie gegebenenfalls halogenierte aliphatische oder aromatische Kohlenwasserstoffe, z.B. Chloroform oder Chlorbenzol, Ether, wie aliphatische, cycloaliphatische oder aromatische Ether, z.B. Diethylether, Dioxan, Diphenylether oder Anisol, Ketone wie aliphatische Ketone, z.B. Aceton oder Methylethylketon, Amide, wie Dialkylamide, z.B. Dimethylformamid, oder Sulfoxide, wie Diniederalkylsulfoxid, z.B. Dimethylsulfoxid.

Ausgangsstoffe der Formel V bzw. Salze davon können nach an sich bekannten Methoden hergestellt werden, beispielsweise durch Umsetzung von Verbindungen der Formel

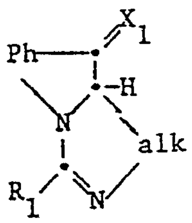

(VIa),

worin $X_1$ Oxo bzw. Thioxo bedeutet, mit Verbindungen der Formel $P(Z_2)_3=C(R_3)-R'_2$ bzw. $X_1=P(Z_3)_2-CH(R_3)-R'_2$, welche sowohl als Phosphonium-Ylide als auch als Phosphorane vorliegen können und worin $X_1$ Oxo oder Thioxo, $Z_2$ Alkyl und/oder Phenyl, $Z_3$ Alkyl und/oder Phenyl bzw. Alkoxy und/oder Phenoxy und $R_3$ Wasserstoff bedeutet. Ausgangsstoffe der Formel V bzw. deren Salze lassen sich z.B. ebenfalls herstellen durch Umsetzung von Verbindungen der Formel

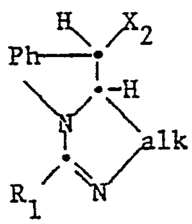

(VIb),

worin $X_2$ eine Gruppe der Formel $-C(R_3)=X'_2$ und $X'_2$ gegebenenfalls funktionell abgewandeltes Oxo bedeutet, mit Cyanwasserstoff oder einem Salz, z.B. einem Alkalimetallsalz, davon. Nach sich gegebenenfalls anschliessender Solvolyse spaltet man aus so erhältlichen Zwischenprodukten der Formel

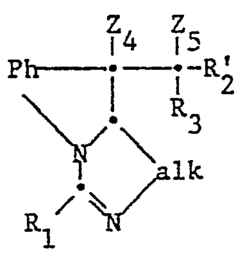

(VII),

worin $Z_4$ gegebenenfalls in Salzform vorliegendes Hydroxy bzw. Thio und $Z_5$ ein Rest der Formel $-P^{\oplus}(Z_2)_3$ bzw. $-P^{\oplus}(Z_3)_2-O^{\ominus}$, $Z_4$ Wasserstoff und $Z_5$ Hydroxy bzw. Mercapto bedeuten, eine Verbindung der Formel $Z_4$-$Z_5$ ab.

Alkoxy ist beispielsweise Niederalkoxy, wie Methoxy, Ethoxy, Propyloxy oder ein Butyloxy. Gegebenenfalls funktionell abgewandeltes Oxo bedeutet Oxo, Thioxo oder gegebenenfalls durch Niederalkyl oder Phenyl substituiertes Imino.

In einer vorteilhaften Ausführungsform des obenstehend beschriebenen Verfahrens zur Herstellung von Verbindungen der Formel I, beispielsweise ausgehend von Verbindungen der Formel VII, kann die Herstellung von Verbindungen der Formel V und die erfindungsgemässe Isomerisierung in situ erfolgen.

Die Abspaltung von $Z_4$-$Z_5$ erfolgt in üblicher Weise, beispielsweise durch Energiezufuhr, z.B. einer Reaktionstemperatur von etwa 50° bis etwa 200° C, oder in Gegenwart eines katalytischen Mittels. Solche sind beispielsweise basische bzw. saure Katalysatoren, wobei als Basen beispielsweise Alkalimetallhydroxide, -amide, -carbonate bzw. -hydride, wie Kaliumhydroxid, Natriumamid, Kaliumcarbonat bzw. Natriumhydrid, Metalloxide, wie Aluminiumoxid, oder organische Stickstoffbasen, wie tertiäre Amine, z.B. Pyridin, Chinolin oder N,N-Dimethylanilin, und als saure Katalysatoren beispielsweise Mineralsäuren, wie Schwefelsäure, Hydrogensulfate, wie Alkalimetallhydrogensulfate, z.B. Kaliumhydrogensulfat, Polyphosphorsäure, Mineralsäureanhydride, wie Phosphorpentoxid, oder Mineralsäurehalogenide, wie Schwefelsäurehalogenide, z.B. Sulfurylchlorid, eingesetzt werden.

Bei der Verfahrensweise zur Bildung von Ausgangsstoffen der Formel V arbeitet man erforderlichenfalls in Gegenwart eines inerten Lösungs- bzw. Verdünnungsmittels, in einem geschlossenen Gefäss und/oder unter Inertgas, z.B. Stickstoff.

Verbindungen der Formeln VIa und VIb ihrerseits lassen sich nach an sich bekannten, analogen Verfahren herstellen, beispielsweise indem man Verbindungen der Formeln

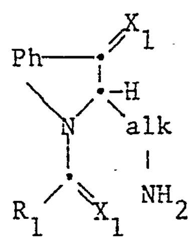

(VId) bzw.

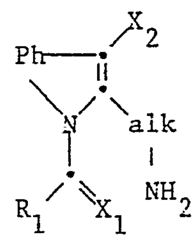

(VIe)

in Gegenwart eines Kondensationsmittels kondensiert. Als Kondensationsmittel eignen sich beispielsweise Säuren, wie Mineralsäuren, z.B. Schwefelsäure, Polyphosphorsäure, Halogenwasserstoff, z.B. Chlorwasserstoffsäure, oder Phosphorsäurehalogenide, wie Phosphoroxychlorid oder Phosphortrichlorid.

Phosphorane der Formel $P(Z_2)_3=C(R_3)-R'_2$ bzw. deren Phosphonium-Ylide können nach an sich beka-nten Methoden hergestellt werden, beispielsweise durch Umsetzung von Phosphinen der Formel $P(Z_2)_3$ mit quartären Ammoniumbasen der Formel $R'_2-CH(R_3-N^{\oplus}(alk')_3B^{\ominus}$, worin alk' ein Alkylrest, wie ein Niederalkylrest, und $B^{\ominus}$ ein Anion, wie Halogenid- oder ein Hydroxylanion, bedeutet, und durch anschliessende Reaktion mit starken Basen, wie Alkalimetalllorganylen, z.B. Butyllithium oder Phenyllithium. Zu den entsprechenden quartären Ammoniumbasen gelangt man ebenfalls durch Reaktion von Phosphinen $P(Z_2)_3$ mit bekannten Verbindungen der Formel $R_2$-Halin Gegenwart von Basen, wie Alkalimetallhydroxiden, -niederalkanolaten, -

hydriden oder -amiden, z.B. Natriumhydroxid, Natriummethylat, Kaliumhydrid oder Kaliumamid.

Verbindungen der Formel $X_1 = P(Z_3)_2\text{-}CH(R_3)\text{-}R'_2$ lassen sichbeispielsweise herstellen, indem man Verbindungen der Formel $P(Z_3)_3$, worin $Z_3$ Alkoxy oder Phenoxy bedeutet, mit Verbindungen der Formel $Hal\text{-}CH(R_3)\text{-}R'_2$, worin Hal Halogen bedeutet, umsetzt.

Verbindungen der Formel VId sind erhältlich durch Acylierung von Verbindungen der Formel

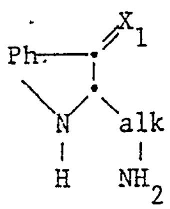

(VIg)

mit Verbindungen der Formel

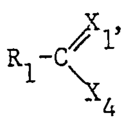

worin $X_4$ Halogen oder Acyl bedeutet.

Acyl leitet sich beispielsweise von einer Carbonsäure, wie Niederalkancarbonsäure, ab und bedeutet beispielsweise Niederalkanoyl, wie Acetyl, Propionyl oder Pivaloyl.

Die Verbindungen der Formel I bzw. Salze davon können ferner hergestellt werden, indem man beispielsweise eine Verbindung der allgemeinen Formel

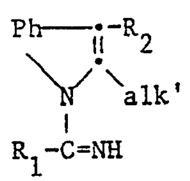

(VIII),

worin alk' eine Gruppe der Formeln $-CH=CH\text{-}Z_6$ oder $CH_2\text{-}CH=Z'_6$ ist, $Z_6$ gegebenenfalls funktionell abgewandeltes Hydroxy oder Amino bzw. $Z'_6$ Oxo oder Imino bedeutet, bzw. ein Salz davon cyclisiert und gewünschtenfalls eine so erhältliche freie Verbindung der Formel I in eine andere freie Verbindung bzw. in ein Salz oder ein verfahrensgemäss erhältliches Salz in die freie Verbindung bzw. in ein anderes Salz überführt.

Funktionell abgewandeltes Hydroxy ist beispielsweise durch Niederalkanol, wie Methanol oder Ethanol, oder einen gegebenenfalls substituierten aromatischen Alkohol, wie Phenol, verethertes Hydroxy, oder mit einem geeigneten Anhydrid, wie Acetanhydrid, mit einer organischen Säure, wie Sulfonsäure, z.B. Niederalkyl- oder gegebenenfalls substituierte Arylsulfonsäure, wie Methansulfonsäure oder p-Toluolsulfonsäure, oder mit einer anorganischen Säure, wie einer Mineralsäure, z.B. Halogenwasserstoffsäure, wie Chlorwasserstoffsäure, verestertes Hydroxy und bedeutet beispielsweise Niederalkoxy, wie Methoxy, oder gegebenenfalls substituiertes Aryloxy, wie Phenoxy, bzw. Niederalkanoyloxy, wie Acetyloxy, Niederalkansulfonyloxy oder gegebenenfalls substituiertes Arylsulfonyloxy, wie Methansulfonyloxy oder p-Toluolsulfonyloxy, oder Halogen, wie Chlor oder Brom.

Die Cyclisierung erfolgt in an sich bekannter Weise, beispielsweise in Gegenwart eines Kondensationsmittels, wie eines sauren Kondensationsmittels. Hierunter fallen beispielsweise Säuren, wie Mineralsäuren, z.B. Schwefelsäure oder Polyphosphorsäure, Mineralsäurehalogenide, wie Phosphorsäurehalogenide, z.B. Phosphoroxichlorid, Phosphortribromid oder Phosphorpentachlorid. Die Reaktion wird erfoderlichenfalls in einem Lösungs- bzw. Verdünnungsmittel bei einem Temperaturbereich von etwa 20° bis etwa 200° C, in einem geschlossenen Gefäss und/oder unter Inertgas, z.B. Stickstoff, durchgeführt.

Inerte Lösungs- bzw. Verdünnungsmittel sind gegebenenfalls substituierte Kohlenwasserstoffe, wie aliphatische oder aromatische Halogenkohlenwasserstoffe, z.B. Chloroform oder Chlorbenzol, gegebenenfalls gemischte Ether, wie aliphatische, cycloaliphatische oder aromatische Ether, z.B. Diethylether, Dioxan, Diphenylether oder Anisol, Ketone wie aliphatische Ketone, wie Aceton oder Methylethylketon, Amide wie Dialkylamide, z.B. Dimethylformamid, oder Sulfoxide, wie Niederalkylsulfoxide, z.B. Dimethylsulfoxid.

Die Ausgangsstoffe der Formel VIII können mittels an sich bekannter Verfahrensweise hergestellt werden, beispielsweise indem man in Verbindungen der Formel

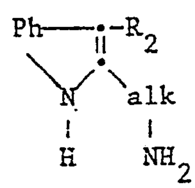

(IX)

die primäre Aminogruppe mittels Alkalimetallnitriten in Gegenwart von Säuren durch Hydroxy substituiert, dieses gegebenenfalls reaktionsfähig verestert, den Indolstickstoff mit einer Verbindung der Formel $R_1\text{-}COOH$ bzw. einem funktionell abgewandelten Derivat davon acyliert und anschliessend mit Ammoniak das entsprechende Amidin bildet.

Die Acylierung des Indolstickstoffs erfolgt nach an sich bekannter Weise, beispielsweise durch Umsetzung mit gegebenenfalls funktionell abgewandelten Carboxy-Derivaten, wie Säuren, Säureanhydriden oder aktivierten Estern. Anhydridisiertes Carboxy ist dabei z.B. mit anorganischen Säuren, wie Halogenwasserstoffsäure, mit

Stickstoffwasserstoffsäure, mit Cyanwasserstoffsäure oder mit organischen Säuren, wie gegebenenfalls durch Halogen substituierte Niederalkansäuren, z.B. Essigsäure, anhydridisiert. Hierunter fallen z.B. Säurehalogenide, z.B. Säurechloride, entsprechende Säureazide, Säurenitrile oder Acyloxycarbonyl.

Die Acylierung mit einer Verbindung der Formel $R_1$-COOH bzw. einem gegebenenfalls funktionell abgewandelten Derivat davon erfolgt in üblicher Weise. Ausgehend von einem Anhydrid, insbesondere einem Säurehalogenid, wird sie bevorzugt in Anwesenheit einer starken Base, beispielsweise eines Alkalimetallhydrids, z.B. Natriumhydrid, eines Alkalimetallamids, z.B. Natriumamid, oder eines Alkalimetallalkoholats, z.B. Kaliummethylat, durchgeführt.

Die Acylierung wie die anschliessende Umsetzung mit Ammoniak wird z.B. in einem inerten Lösungsmittel vorgenommen, wie in einem alkylierten Amid, z.B. N,N-Dimethylformamid, einem gegebenenfalls halogenierten Kohlenwasserstoff, z.B. Chloroform oder Chlorbenzol, oder einem Nitril, z.B. Acetonitril, oder einer Mischung davon, erforderlichenfalls bei erniedrigter oder erhöhter Temperatur und/oder in einer Inertgasatmosphäre.

Die Verbindungen der Formel IX lassen sich ihrerseits nach an sich bekannten Verfahren herstellen, beispielsweise analog der Fischer'schen Indolsynthese durch Behandlung von Phenylhydrazonen bzw. entsprechend 1,3-substituierten 4-Piperidone mit Säuren, wie mit äthanolischer Salzsäure, oder durch Acylierung und Kondensation von entsprechend substituierten α-Hydroxyketonen mit gegebenenfalls substituierten Anilinen.

Die Verbindungen der Formel IX lassen sich ausserdem in einer vorzugsweisen Ausführungsfrom herstellen, indem man beispielsweise von Verbindungen der Formel

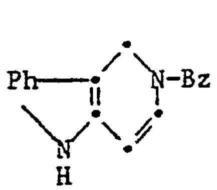

(IVd)

ausgeht, worin Bz ein gegebenenfalls substituierter α-Phenylniederal-kylrest, vorzugsweise Benzyl, bedeutet, insbesondere mit Benzylbromid, quaternisiert und mittels eines Nucleophils, vorzugsweise mit Cyaniden, die Bindung am so erhaltenen quartären Stickstoff spaltet und in einer als Zwischenstufe erhältlichen Verbindung der Formel

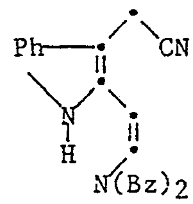

(IVe)

die Cyanogruppe wie gewünscht solvolysiert und die Benzylgruppen beispielsweise hydrogenolytisch in Gegenwart eines Hydrierungskatalysators, z.B. von Palladium, abspaltet.

Die Verbindungen der allgemeinen Formel I bzw. Salze davon sind ferner herstellbar, indem man beispielsweise in einer Verbindung der Formel

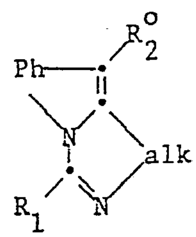

(X),

worin $R^o_2$ einen in die Gruppe $R_2$ überführbaren Rest bedeutet, oder Salzen davon $R^o_2$ solvolytisch oder oxidativ in die Gruppe $R_2$ überführt, und gewünschtenfalls eine so erhältliche freie Verbindung der Formel I in eine andere freie Verbindung bzw. in ein Salz oder ein verfahrensgemäss erhältliches Salz in die freie Verbindung bzw. in ein anderes Salz überführt.

So ist beispielsweise $R^o_2$ eine Gruppe der Formel $-CH(R_3)-R''_2$, in der $R_3$ Wasserstoff und $R''_2$ von $R'_2$ verschiedenes funktionell abgewandeltes Carboxy, eine Gruppe der Formel $-C=O)-N^{\oplus}{}_2B^{\ominus}$, wobei $B^{\ominus}$ das Anion einer Mineralsäure, z.B. Chlorid,Bromid, oder Tetrafluoroborat, und $R'_2$ mit dem eingangs beschriebenen Carboxy- bzw. Niederalkoxycarbonyl-Teil von $R_2$ identisch ist, oder gegebenenfalls bis zur Stufe des Formyl oxidiertes Methyl bedeutet.

Funktionell abgewandelte und von $R'_2$ verschiedene, funktionell abgewandelte Carboxy-Reste sind beispielsweise entsprechendes, verestertes oder amidiertes Carboxy, gegebenenfalls funktionell abgewandelte Orthoestergruppen, wie Trihalogen-, Halogendiniederalkoxy- bzw. Triniederalkoxymethylgruppen, anhydridisiertes Carboxy, wie Cyano, eine Gruppe der Formel $=C=O$, Cyano-, Azido- oder Halogencarbonyl, Acyloxycarbonyl, Niederalkanoyl-, wie z.B. Acetyloxycarbonyl, oder Derivate von Carboxy der Formel $R'_1$ oder $R''_2$, in denen Oxo gegebenenfalls durch Thio bzw. gegebenenfalls substituiertes Imino ersetzt ist, wie gegebenenfalls verestertes Thiocarboxy, wie Niederalkylthiocarboxy, z.B. Ethylthiocarboxy,

amidiertes Thiocarboxyl, Iminoester, wie Imid- bzw. Amidhalogenidgruppierungen, z.B. Iminochlor- oder Aminodichlormethyl, Iminoethergruppierungen, wie Niederalkyl- oder Niederalkyleniminoethergruppierungen, z.B. Methoxy- oder Ethoxyiminomethylen, oder Amidinogruppen, wie Amidino oder Niederalkyl-, z.B. Methylamidino.

Verestertes Carboxy $R''_2$ ist beispielsweise Niederalkoxycarbonyl, welches durch gegebenenfalls substituiertes Aryl, wie Phenyl oder Pyridyl, einfach oder durch Hydroxy, Halogen bzw. Niederalkoxy ein- oder mehrfach substituiert sein kann, wie durch Hydroxy, Niederalkoxy und/oder Halogen substituiertes Niederalkoxycarbonyl, z.B. Mono- bzw. Dihydroxyniederalkoxy-, Halogen- oder Niederalkoxyniederalkoxycarbonyl, oder durch Niederalkyl, Niederalkoxy und/oder Halogen substituiertes Phenylniederalkoxycarbonyl.

Amidiertes Carboxy $R''_2$ ist beispielsweise Carbamoyl, welches gegebenenfalls durch Hydroxy bzw. Amino einfach, durch Niederalkyl bzw. Hydroxyniederalkyl bzw. Phenylniederalkyl ein- bzw. zweifach oder durch 4- bis 7-gliedriges Niederalkylen bzw. 3-Oxa-, 3-Thia- oder 3-Azaalkylen zwei fach substituiert sein kann. Als Beispiele seien genannt: Carbamoyl, N-Hydroxy-, N-Amino- N-Mono- bzw. N N-Diniederalkyl- oder N-Mono- bzw. N,N-Dihydroxyalkylcarbamoyl. Durch 4- bis 7-gliedriges Niederalkylen zweifach N-substituiertes Carbamoyl ist z.B. Pyrrolidino- oder Piperidinocarbonyl bzw. Morpholino-, Thiomorpholino-, Piperazino- oder N-Niederalkyl-, wie N-Methylpiperazino-carbonyl.

Bis zur Stufe des Formyl oxidiertes Methyl bzw. funktionell abgewandelte Gruppen davon sind beispielsweise gegebenenfalls reaktionsfähig verestertes oder verethertes Hydroxymethyl bzw. gegebenenfalls funktionell abgewandeltes Formyl, wie Hydroxymethyl, Mono- oder Dihalogenmethyl, Niederalkoxymethyl, Formyl oder Formimino.

Funktionell abgewandelte Carboxy-Verbindungen, wie verestertes oder amidiertes Carboxy, gegebenenfalls funktionell abgewandelte Orthoester, anhydridisiertes Carboxy oder Acyloxycarbonyl, lassen sich direkt oder in mehreren Solvolyseschritten zu freiem oder verestertem Carboxy $R'_2$ solvolysieren. Von $R'_2$ verschiedenes verestertes Carboxy kann durch übliche Umesterung in verestertes Carboxy $R'_2$ überführt werden.

Die Solvolyse von $R''_2$ erfolgt in bekannter Weise, beispielsweise durch Hydrolyse mit Wasser oder durch Alkoholyse, z.B. von Cyano oder gegebenenfalls substituiertem Carbamoyl, zu verestertem Carboxy $R'_2$ mit einem entsprechenden Alkohol. Auch die Umesterung erfolgt durch Alkoholyse mit dem gewünschten Alkohol. Dabei wird jeweils, erforderlichenfalls in Gegenwart eines Katalysators, in einem Lösungs- bzw. Verdünnungsmittel, in einem geschlossenen Gefäss, in einem Temperaturbereich von etwa 0° bis etwa 150°C, und/oder unter Inertgas, z.B. Stickstoff, gearbeitet.

Katalysatoren sind beispielsweise basische Kondensationsmittel, wie Alkalimetall- oder Erdalkalimetallhydroxide, z.B. Natrium-, Kaliumoder Calciumhydroxid, oder tertiäre organische Amine, wie Pyridin oder Trialkylamine, z.B. Triethylamin, oder saure Hydrolysemittel, wie Mineralsäuren, z.B. Halogenwasserstoffsäuren, wie Chlorwasserstoffsäure, oder organische Carbon- oder Sulfonsäuren, wie Niederalkancarbonsäuren oder gegebenenfalls substituierte Benzolsulfonsäuren, z.B. Essigsäure oder p-Toluolsulfonsäure.

Gegebenenfalls bis zur Stufe des Formyl oxidiertes Methyl, wie Methyl, Hydroxymethyl oder Formyl, bzw. funktionell abgewandelte Derivate davon, wie Halogenmethyl, z-B. Chlormethyl, Mercaptomethyl, Thioformyl oder gegebenenfalls substituiertes Formimino, lassen sich beispielsweise direkt oder über mehrere Oxidationsschritte, gegebenenfalls über Hydroxymethyl oder Formyl, zu Carboxy oxidieren. Verethertes Hydroxymethyl, vorzugsweise Niederalkoxymethyl, z.B. Ethoxymethyl, wird in Gegenwart eines Oxidationsmittels zu Niederalkoxycarbonyl oxidiert. Die Umsetzung von Formyl zu Carbamoyl wird beispielsweise mit Hilfe einer Aminoverbindung in Gegenwart eines Oxidationsmittels, wie eines Uebergangsmetalloxides, z.B. Mangandioxid, und erfoderlichenfalls in Gegenwart eines Nucleophils, insbesondere eines Cyanids, durchgeführt.

Oxidation von $R''_2$ wird auf üblichem Wege durchgeführt, beispielsweise unter Verwendung der üblichen Oxidationsmittel. Solche sind z.B. gegebenenfalls katalytisch angeregter Sauerstoff, Alkalimetallsalze von Chromaten bzw. Manganaten, wie Natriumchromat oder Kaliumpermanganat, oder Uebergangsmetalloxide, wie Mangandioxid oder Chromtrioxid. Dabei arbeitet man erforderlichenfalls in einem inerten Lösungsmittel, in einem geschlossenen Gefäss und/oder unter Kühlen oder Erwärmen, z.B. bei etwa 0° bis etwa 150°C.

Die Ausgangsstoffe der Formel X können nach analogen Verfahren hergestellt werden, beispielsweise indem man Verbindungen der Formel

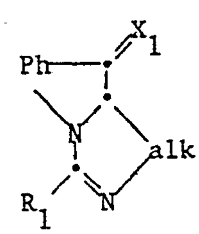

(VIa),

worin $X_1$ Oxo bzw. Thioxo bedeutet, mit Verbindungen der Formel $P(Z_2)_3\text{-}R^o_2$, welche sowohl als Phosphonium-Ylide als auch als Phophorane vorliegen können, bzw. $X_1 = P(Z_3)_2$-

$R^o_2$, und worin $Z_2$ Alkyl und/oder Phenyl und $Z_3$ Alkyl und/oder Phenyl bzw. Alkoxy, wie Niederalkoxy, und/oder Phenoxy und R'' von R' verschiedenes funktionell abgewandeltes Carboxy, eine Gruppe der Formel -$C(=O)_N{}^{\oplus}{}_{2B}{}^{\ominus}$ oder gegebenenfalls bis zur Stufe des Formyl oxidiertes Methyl bedeutet, umsetzt, aus gegebenenfalls so erhältlichen Zwischenprodukten der Formel

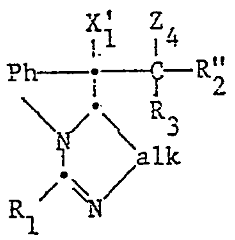

(VIh),

worin $X'_1$ $-O^{\ominus}$ bzw. $-S^{\ominus}$ und $Z_4$ einen Rest der Formel $-^{\oplus}P(Z_2)_3$ bzw. $-P^{\oplus}(X_1')(Z_3)_2$ bedeutet, eine Verbindung der Formel $X_1=P(Z_2)_3$ bzw. $X_1=P(X_1')(Z_3)_2$ abspaltet und eine so erhaltene Verbindung zu einer Verbindung der Formel X isoliert.

Die Umsetzung erfolgt üblicherweise in einem inerten Lösungsmittel, beispielsweise einem gegebenenfalls halogenierten Kohlenwasserstoff, wie einem Aromaten, z.B. Benzol oder Toluol, einem Ether, wie Tetrahydrofuran oder Dioxan, oder einem Amid, z.B. Dimethylformamid, in einem Temperaturbereich von etwa 20° bis etwa 150°C und/oder gegebenenfalls in Gegenwart eines Katalysators, wie einer Base, z.B. eines Alkalimetallalkoholats, wie Kalium-tert.-butylat.

Insbesondere gelangt man zu den Ausgangsstoffen der Formel X worin $R_2^o$ ein solvolytisch oder oxidativ in $R_2$ überführbarer Rest ist, und geht beispielsweise von Verbindungen der Formel

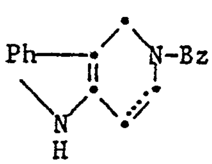

(IVd)

aus, worin Bz ein gegebenenfalls substituierter α-Phenylniederalkyl-rest, vorzugsweise Benzyl, bedeutet, quaterniert das tertiäre Stickstoffatom, insbesondere mit Benzylchlorid, spaltet die Bindung am quartären Stickstoffatom mit Hilfe einer starken Base, wie mit Cyaniden, z.B. Natriumcyanid, und überführt in einer so erhaltenen Verbindung der Formel

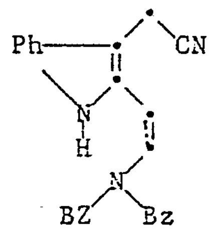

(IVe)

die Cyanogruppe in $R^o_2$, beispielsweise durch Solvolyse zu Carboxy oder Niederalkoxycarbonyl und anschliessender Reduktion zu Hydroxymethyl bzw. Niederalkoxymethyl, spaltet die Bz-Gruppen hydrogenolytisch in Gegenwart eines Hydrierungskatalysators ab. Anschliessend setzt man die so erhaltene Verbindung mit einer Verbindung der Formel

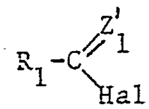

,

worin $Z'_1$ gegebenenfalls funktionell abgewandeltes Oxo und Hal Halogen bedeutet, um und führt die Cyclisierung zu entsprechenden Verbindungen der Formel X in Gegenwart eines üblichen Cyclisierungsmittels, wie Mineralsäurehalogenids, z.B. Phosphoroxychlorid, durch. In einer bevorzugten Ausführungsform dieser Verfahrensvariante bildet man zunächst, ausgehend von entsprechenden Ausgangsstoffen der Formeln IVd, IVe und $R_1$-$C(=Z'_1)$-Hal, ohne Isolierung von Zwischenprodukten solche Verbindungen der Formel X, worin alk 1,2-Ethylen ist und $R_1$, $R^o$ sowie Ph die angegebenen Bedeutungen haben.

Insbesondere werden entsprechende Verbindungen der Formel X, worin alk 1,2-Ethylen ist und $R^o_2$ Cyanomethyl oder daraus gebildetes, gegebenenfalls substituiertes Carbamoylmethyl bedeutet zunächst analog der nachstehend beschriebenen Dehydrierung dehydriert und anschliessend wie gewünscht zu den Zielverbindungen der Formel I solvolysiert.

Entsprechend werden unter Verwendung geeigneter Ausgangsstoffe der Formeln IVd, IVe und $R_1$-$C(=Z'_1)$-Hal ohne Isolierung von Zwischenprodukten solche 3,4-Dihydroverbindungen der Formel X gebildet, worin $R_2$ Niederalkoxycarbonylmethyl bedeutet und alk 1,2-Ethylen ist. Die Niederalkoxycarbonylgruppe wird im nächsten Reaktionsschritt zu 2-Hydroxy-ethyl $R^o_2$ reduziert, beispielsweise unter Verwendung komplexer Hydride, wie Lithiumaluminiumhydrid, und die Hydroxygruppe mit einem gewünschten Niederalkanol verethert. Anschliessend werden die entsprechend erhältlichen Verbindungen in der nachstehend beschriebenen Art dehydriert. 2-Niederalkoxyethyl $R^o_2$, daraus gebildetes 2-Hydroxyethyl sowie Formylmethyl $R^o_2$ werden schliesslich wie vorstehend angegeben in der gewünschten Weise zu $R_2$ oxidiert.

Die Verbindungen der Formel I werden in erster Linie hergestellt, indem man entsprechende Verbindungen der Formel

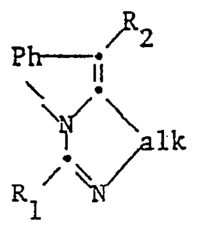

(I),

worin alk Ethylen bedeutet unter Abspaltung von Wasserstoff bei gleichzeitiger Bildung einer zusätzlichen Bindung dehydriert und gewünschtenfalls eine erfindungsgemäss erhältliche Verbindung in eine andere Verbindung der Formel I bzw. eine erfindungsgemäss erhältliche freie Verbindung der Formel I in ein Salz oder ein verfahrensgemäss erhältliches Saiz in die freie Verbindung der Formel I bzw. in ein anderes Salz überführt.

Die Dehydrierung erfolgt in an sich bekannter Weise, insbesondere bei erhöhter Temperatur, beispielsweise in einem Temperaturintervall von Raumtemperatur, insbesondere von etwa 100° bis etwa 300° C, und unter Verwendung eines Dehydrierungsmittels. Als solche Mittel kommen beispielsweise Dehydrierungskatalysatoren, z.B. Nebengruppenelemente, vorzugsweise der Nebengruppe VIII, wie Palladium oder Platin, oder deren Salze, wie Ruthenium-triphenylphosphidchlorid, in Betracht, wobei die Katalysatoren gegebenenfalls auf geeignetem Trägermaterial, wie Kohle, Aluminiumoxid oder Siliziumdioxid, aufgezogen sind. Weitere Dehydrierungsmittel sind beispielsweise Chinone, wie p-Benzochinone, z.B. Tetrachlorp-benzochinon oder 2,3-Dichlor-5,6-dicyano-p-benzochinon, oder wie Anthrachinone,z.B. Phenanthren-9,10-chinon. Bevorzugte Dehydrierungskatalysatoren sind geeignete Selen-Derivate, insbesondere Selendioxid oder Diphenylselenium-bis(trifluoroacetat), ferner Diphenyl-selenoxid. In einer vorteilhaften Ausführungsform der vorstehend beschriebenen Dehydrierung wird dieser Prozess bei Verwendung von Selendioxid als Katalysator bei erhöhter Temperatur durchgeführt, bei Verwendung von Diphenylseleniumbis-(trifluoracetat) erfolgt die Dehydrierung bei Raumtemperatur.

Die Umsetzung wird in einem inerten, gegebenenfalls hochsiedenden Lösungsmittel, wie einem Ether, z.B. Diphenylether, erforderlichenfalls unter Druck, in einem geschlossenen Gefäss und/oder unter Inertgas, z.B. Stickstoff durchgeführt.

Die Herstellung von Ausgangsstoffen der Formel I, worin alk 1,2-Ethylen bedeutet, erfolgt analog der eingangs beschriebenen Ausführungsweise, wobei man von Verbindungen der Formeln IVd ausgeht, diese beispielsweise mit Benzylhalogenidem erschöpfend alkyliert und die Bindung am quartären N-Atom mit Alkalimetallcyaniden spaltet. In den so erhältlichen Verbindungen der Formel IVe kann die Cyanogruppe in üblicher Weise wie gewünscht solvolysiert werden, dabei wird die Ueberführung in Niederalkoxycarbonyl bevorzugt. Nach hydrogenolytischer Abspaltung der Benzylgruppen erfolgt die Umsetzung mit Verbindungen der Formel $R_1$-C(=Z'$_1$)-Hal, wobei mit Hilfe von Cyclisierungsmitteln, wie Phosphoroxychlorid, unter den Reaktionsbedingungen entsprechende Verbindungen der Formel II gebildet werden, die direkt zu entsprechenden Verbindungen der Formel I führen, worin alk 1,2-Ethylen und $R_2$ vorzugsweise Niederalkoxycarbonylmethyl bedeutet.

Wird im Anschluss an die nachfolgende Dehydrierung die Herstellung von freien Säure-Derivaten gewünscht werden erhaltene Niederalkylester vorteilhaft hydrolysiert.

Eine erfindungsgemäss erhältliche Verbindung der Formel I kann in an sich bekannter Weise in eine andere Verbindung der Formel I umgewandelt werden.

Enthält die Gruppe $R_2$ ein freies Carboxy so lässt sich dieses nach an sich bekannten Veresterungsmethoden in entsprechend verestertes Carboxy uberführen, beispielsweise indem man gegebenenfalls reaktionsfähiges abgewandeltes Carboxy oder ein Salz davon durch Alkoholyse mit einem gewunschten Alkohol, beispielsweise einem reaktionsfähigen Derivat davon oder einem davon abgeleiteten Olefin, umsetzt oder durch Alkylierung mit Diazoniederalkan.

Geeignete reaktionsfähige funktionelle Carboxyderivate sind beispielsweise Anhydride, wobei als Anhydride insbesondere gemischte Anhydride, z.B. solche mit anorganischen Säuren, wie Halogenwasserstoffsauren, z.B. Chlorwasserstoffsäure wie Stickstoffwasserstoff-oder Cyanwasserstoffsäuren, oder mit organischen Carbonsäuren, wie Niederalkansäuren, z.B. Essigsäure, verwendet werden.

Reaktionsfähige Derivate eines Alkohols sind beispielsweise Carbon-, Phosphorig- Schweflig- oder Kohlensäureester, z.B. Niederalkancarbonsäureester, Triniederalkylphosphit, Diniederalkylsulfit oder Pyrocarbonat, oder Mineral- bzw. Sulfonsäureester, z.B. Chlor-, Brom- oder Schwefelsäureester, Benzol-, Toluol- oder Methansulfonsäureester, des betreffenden Alkohols.

Die Veresterung von freiem Carboxy wird in Gegenwart eines Kondensationsmittels durchgeführt. Als katalytisch wasserabspaltende Mittel für die Veresterung mit Alkoholen kommen z.B. Säuren, beispielsweise Protonsäuren, wie Chlorwasserstoff-, Bromwasserstoff-, Schwefel-, Phosphor-, Bor-, Benzolsulfon- und/oder Toluolsulfonsäurc, bzw. Lewissäuren, wie Bortrifluorid-Etherat, in Frage. Uebliche wasserbindende Kondensationsmittel sind z'B'

durch Kohlenwasserstoffreste substituierte Carbodiimide, beispielsweise N,N'-Diethyl-, N,N-Dicyclohexyl- oder N-Ethyl-N'-(3-dimethylaminopropyl)-carbodiimide. Kondensationsmittel für die Veresterung mit reaktionsfähigen Estern sind basische Kondensationsmittel, wie anorganische Basen, z.B. Alkalimetall- oder Erdalkalimetallhydroxide bzw. -carbonate, wie Natrium-, Kalium- oder Calciumhydroxid bzw. -carbonat, wie organische Stickstoffbasen, z.B. tertiäre organische Amine, wie Triethylamin oder Pyridin. Die Veresterung wird vorteilhaft in einem Ueberschuss an eingesetztem Alkohol durchgeführt. Dabei arbeitet man vorzugsweise in einem wasserfreien Medium, erforderlichenfalls in Gegenwart eines inerten Lösungsmittels, wie in halogenierten Kohlenwasserstoffen, z.B. Chloroform oder Chlorbenzol, in Ethern, z.B. Tetrahydrofuran oder Dioxan.

Die Umsetzung mit einem Olefin kann beispielsweise in Gegenwart eines sauren Katalysators, z.B. einer Lewissäure, z.B. von Bortrifluorid, einer Sulfonsäure, z.B. von p-Toluolsulfonsäure, oder vor allem eines basischen Katalysators, z.B. von Natrium- oder Kaliumhydroxid, vorteilhaft in einem inerten Lösungsmittel, wie einem Ether, z.B. in Diethylether oder Tetrahydrofuran, erfolgen.

Weiterhin kann man freies Carboxy bzw. reaktionsfähig funktionelle Carboxyderivate durch Solvolyse mit Ammoniak oder einem primären bzw. sekundären Amin, wobei auch Hydroxylamin bzw. Hydrazine eingesetzt werden können, in üblicher Weise unter Dehydratisierung, gegebenenfalls in Anwesenheit eines Kondensationsmittels,in eine gewünschte amidierte Form überführen. Als Kondensationsmittel werden vorzugsweise Basen verwendet, beispielsweise anorganische Basen, wie Alkalimetallhydroxide, z.B. Natrium- oder Kaliumhydroxid, organische Stickstoffbasen, wie tert.-Amine, z.B. Pyridin, Tributylamin oder N-Dimethylanilin, oder Tetrahalogensilane, wie Tetrachlorsilan.

Ferner kann man erfindungsgemäss erhältliche Verbindungen der Formel I worin $R_2$ als Substituenten verestertes Carboxy enthält, in üblicher Weise umestern beispielsweise durch Reaktion mit einem entsprechenden Alkohol bzw. einem Metallsalz davon, wie einem Alkalimetallsalz, z.B. dem Natrium- oder Kaliumsalz, erforderlichenfalls in Gegenwart eines Katalysators, beispielsweise einer starken Base, wie eines Alkalimetallhydroxides, -amides oder -alkoholates, z.B. Kaliumhydroxid, Natriumamid oder -methylat, oder einer starken Säure, wie einer Mineralsäure, z.B. Schwefelsäure, Phosphorsäure oder Salzsäure, oder wie einer organischen Sulfonsäure, z.B. einer aromatischen Sulfonsäure, wie p-Toluolsulfonsäure.

Verestertes Carboxy kann weiterhin nach bekannten Verfahren, z.B. durch Hydrolyse in Gegenwart eines Katalysators, in die freie Carboxygruppe überführt werden. Als Katalysatoren kommen vorzugsweise Basen, z.B. Alkalimetallhydroxide, wie Natrium- oder Kaliumhydroxid, in Frage. Verestertes Carboxy lässt sich ferner in üblicher Weise, beispielsweise durch Solvolyse, gegebenenfalls in Gegenwart eines Katalysators, beispielsweise eines sauren bzw. basischen Mittels, in Carboxy, durch Ammonolyse oder Aminolyse mit Ammoniak oder mit einem primären bzw. sekundären Amin, in amidiertes Carboxy umwandeln. Als Basen finden beispielsweise Alkalimetallhydroxide, wie Natrium- bzw. Kaliumhydroxide, und als Säuren beispielsweise Mineralsäuren, wie Schwefelsäure, Phosphorsäure oder Salzsäure, Verwendung. Ebenso kann man erfindungsgemäss erhältliche Verbindungen der Formel I, worin die Gruppe $R_2$ den Substituenten amidiertes Carboxy enthält, nach an sich bekannten Methoden die Amidbindung spalten und so das Carbamoyl in freies Carboxy überführen. Hierzu arbeitet man in Gegenwart eines Katalysators, beispielsweise einer Base, wie eines Alkalimetall- oder Erdalkalimetallhydroxides oder -carbonates, z.B. Natrium-, Kalium- oder Calciumhydroxid oder -carbonat, oder einer Säure, wie einer Mineralsäure, z.B. Salzsäure, Schwefelsäure oder Phosphorsäure.

Enthält die Gruppe $R_2$ der Formel I eine veresterte Carboxygruppe, so kann man diese beispielsweise durch übliche Solvolyse, vorteilhaft durch einen Ueberschuss an Ammoniak oder mindestens ein Wasserstoffatom enthaltendes Amin, gegebenenfalls in Gegenwart eines Katalysators, in eine amidierte Carboxygruppe überführen. Dabei verwendet man als Katalysatoren beispielsweise Säuren, wie Mineralsäuren, z.B. Salz-, Schwefel- oder Phosphorsäure, oder Basen, wie Alkalimetallhydroxide, z.B. Natrium- oder Kaliumhydroxid.

Enthält die Gruppe $R_2$ der Formel I als Substituenten amidiertes Carboxy, so kann man dieses beispielsweise durch übliche Solvolyse mit einem Alkohol in Gegenwart eines Katalysators in verestertes Carboxy überführen. Dabei verwendet man beispielsweise saure Katalysatoren, wie Mineralsäuren, z.B. Phosphorsäure, Salzsäure oder Schwefelsäure.

Falls der Substituent $R_1$ der Formel I durch Niederalkylthio substituiert ist, kann man diesen auf übliche Weise zu entsprechendem Niederalkansulfinyl bzw. -sulfonyl oxidieren. Als geeignetes Oxidationsmittel Perschwefelsäure, organische Persäuren, wie entsprechende Percarbonoer Persulfonsäuren, z.B. Perameisen-, Peressig-, Trifluorperessigbzw. Perbenzoesäure oder p-Toluolpersulfonsäure, oder Gemische aus Wasserstoffperoxid und Säuren, z.B. Gemisch aus Wasserstoffperoxid mit Essigsäure, in Betracht.

Häufig führt man die Oxidation in Gegenwart von geeigneten Katalysatoren durch, wobei als Katalysatoren geeignete Säuren, wie gegebenenfalls substituierte Carbonsäuren, z.B.

Essigsäure oder Trifluoressigsäure, oder Uebergangsmetalloxide, wie Oxide von Elementen der VII. Nebengruppe, z.B. Vanadium-, Molybdän- oder Wolframoxid, zu nennen sind. Die Oxidation wird unter milden Bedingungen, z.B. bei Temperaturen von etwa −50° bis etwa +100° C, durchgeführt.

Die Oxidation zur Sulfonstufe kann man auch mit Distickstofftetroxid als Katalysator in Gegenwart von Sauerstoff bei tiefen Temperaturen entsprechend durchführen, ebenso wie die direkte Oxidation des Niederalkylthio zum Niederalkansulfonyl. Jedoch setzt man hierbei üblicherweise das Oxidationsmittel im Ueberschuss ein.

Verbindungen der Formel I, worin $R_1$ einen durch Niederalkylsulfinyl bzw. -sulfonyl substituierten aromatischen Rest darstellt, lassen sich nach an sich bekannten Methoden zu den entsprechenden Niederalkylthio-Verbindungen, von Niederalkansulfonyl-Derivaten ausgehend, auch zu Niederalkan-sulfinyl reduzieren. Als Reduktionsmittel eignen sich beispielsweise katalytisch aktivierter Wasserstoff, wobei Edelmetalle bzw. Oxide, wie Palladium, Platin oder Rhodium bzw. deren Oxide, verwendet werden, gegebenenfalls auf geeignetem Trägermaterial, wie Aktivkohle oder Bariumsulfat, aufgezogen. Weiterhin kommen reduzierende Metallkationen, wie Zinn II-, Blei II-, Kupfer I-, Mangan II-, Titan II-m Vanadium II-, Molybdän III- oder Wolfram III-Verbindungen, Halogenwasserstoffe, wie Chlor-, Brom- oder Jodwasserstoff, Hydride, wie komplexe Metallhydride, z.B. Lithiumaluminium-, Natriumbor-, Tributylzinnhydrid, Phosphorverbindungen, wie Phosphorhalogenide, z.B. Phosphortrichlorid, -bromid, Phosphorpentachlorid oder Phosphoroxichlorid, Phosphine, wie Triphenylphosphin, oder Phosphorpentasulfid-Pyridin, oder Schwefelverbindungen, wie Mercaptane, Thiosäuren, wie Thiophosphorsäuren oder Dithiocarbonsäuren, Dithionit oder Schwefel-Sauerstoff-Komplexe, wie Jod-Pyridin-Schwefeldioxidkomplex, in Frage.

Erhaltene Salze können in an sich bekannter Weise in die freien Verbindungen umgewandelt werden, z.B. durch Behandeln mit einem sauren Reagens, wie einer Mineralsäure, bzw. einer Base, z.B. Alkalilauge.

Die neuen Verbindungen können, je nach der Wahl der Ausgangsstoffe und Arbeitsweisen, in Form eines der möglichen Isomeren oder als Gemische derselben, z.B. je nach der Anzahl der asymmetrischen Kohlenstoffatome, als reine optische Isomere, wie Antipoden oder als Isomerengemische, wie Racemate, Diastereoisomerengemische oder Racematgemische, vorliegen.

Erhaltene Diastereomerengemische und Racematgemische können auf Grund der physikalisch-chemischen Unterschiede der Bestandteile in bekannter Weise in die reinen Isomeren, Diastereomeren oder Racemate aufgetrennt werden, beispielsweise durch Chromatographie und/oder fraktionierte Kristallisation.

Erhaltene Racemate lassen sich ferner nach bekannten Methoden in die optischen Antipoden zerlegen, beispielsweise durch Umkristallisation aus einem optisch aktiven Lösungsmittel, mit Hilfe von Mikroorganismen oder durch Umsetzung eines sauren Endstoffes mit einer mit der racemischen Säure Salze bildenden optisch aktiven Base und Trennung der auf diese Weise erhaltenen Salze, z.B. auf Grund ihrer verschiedenen Löslichkeiten, in die Diastereomeren, aus denen die Antipoden durch Einwirkung geeigneter Mittel freigesetzt werden können, zerlegt. Vorteilhaft isoliert man den wirksameren der beiden Antipoden.

Die Verbindungen einschliesslich ihrer Salze können auch in Form ihrer Hydrate erhalten werden oder andere zur Kristallisation verwendete Lösungsmittel einschliessen.

Infolge der engen Beziehung zwischen den neuen Verbindungen in freier Form und in Form ihrer Salze sind im vorausgegangenen und nachfolgend unter den freien Verbindungen oder ihren Salzen sinn- und zweckgemäss gegebenenfalls auch die entsprechenden Salze bzw. freien Verbindungen zu verstehen.

Die Erfindung betrifft auch diejenigen Ausführunsformen des Verfahrens, nach denen man von einer auf irgendeiner Stufe des Verfahrens als Zwischenprodukt erhältlichen Verbindungen ausgeht und die fehlenden Schritte durchführt oder einen Ausgangsstoff in Form eines Salzes verwendet oder insbesondere unter den Reaktionsbedingungen bildet.

Beim Verfahren der vorliegenden Erfindung werden vorzugsweise solche Ausgangsstoffe verwendet, welche zu den eingangs als besonders wertvoll geschilderten Verbindungen führen. Neue Ausgangsstoffe und Verfahren zu ihrer Herstellung bilden ebenfalls einen Gegenstand der Erfindung.

Bei den erfindungsgemässen pharmazeutischen Präparaten, welche Verbindungen der Formel I oder pharmazeutisch verwendbare Salze davon enthalten, handelt es sich um solche zur enteralen, wie oralen oder rektalen, und parenteralen Verabreichung sowie zur topischen Anwendung an Warmblüter(n), welche den pharmakologischen Wirkstoff allein oder zusammen mit einem pharmazeutisch anwendbaren Trägermaterial enthalten. Die Dosierung des Wirkstoffs hängt von den Warmblüter-Spezies, dem Alter und dem individuellen Zustand, sowie von der Applikationsweise ab. Im Normalfall ist für einen etwa 75 kg schweren Warmblüter bei oraler Applikation eine ungefähre Tagesdosis von etwa 30-300mg, vorteilhaft in mehreren gleichen Teildosen verteilt, zu veranschlagen.

Die neuen pharmazeutischen Präparate enthalten z.B. von etwa 10% bis etwa 80%, vorzugsweise von etwa 20% bis etwa 60% des Wirkstoffs. Erfindungegemässe pharmazeutische

Präparate zur enteralen bzw. parenteralen Verabreichung sind z.B. solche in Dosiseinheitsform, wie Dragees, Tabletten, Kapseln oder Suppositorien, ferner Ampullen. Diese werden in an sich bekannter Weise, z.B. mittel konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragee-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder CalciumHydrogenphosphat, ferner Bindemittel wie Stärkekleister unter Verwendung von z.B. Mais-, Weizen- Reis- oder Kartoffelstärke Gelatine, Tragakanth, Methylcellulose und/oder Polyvinylpyrrolidon, und/oder wenn erwünscht, Sprengmitteln, wie die obegenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliess-, Regulierund Schmiermittel, z.B. Kieselsäure, Talk, Stearinsäure oder Salze davon, wie Magnesium- oder Calciumstearat, und/oder Polyethylenglykol. Dragee-Kerne werden mit geeigneten, gegebenenfalls Magensaft-resistenten Ueberzügen versehen, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragee-Ueberzügen können Farbstoffe oder Pigmente, z.B. zur Identifierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken und/oder Gleitmitteln, wie Talk oder Magnesiumstearat, und gegebenenfalls von Stabilisatoren, enthalten. In weichen Kapseln ist der Wirkstoff vorzugsweise in geeigneten Flussigkeiten, wie fetten Oelen, Paraffinöl oder flüssigen Polyethylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffes mit einer Suppositoriengrundmasse bestehen. Als Suppositoriengrundmasse einen sich z.B. naturliche oder synthetische Triglyceride, Paraffin kohlenwasserstoffe Polyethylenglykole oder höhere Alkanole. Ferner können auch Gelatine-Rektalkapseln verwendet werden, die eine Kombination des Wirkstoffs mit einer Grundmasse enthalten; als Grundmassenstoffe kommen z.B. flüssige Triglyceride, Polyethylengylkole oder Paraffinkohlenwasserstoffe in Frage.

Zur parenteralen Verabreichung eignen sich in erster Linie wässrige Lösungen eines Wirkstoffs in wasserlöslicher Form, z.B. eines wasserlöslichen Salzes, ferner Suspensionen des Wirkstoffes, wie entsprechende ölige Injektionssuspensionen, wobei man geeignete lipophile Lösungsmittel oder Vehikel, wie fette Oele, z.B. Sesamöl, oder synthetische Fettsäureester, z.B. Ethyloleat oder Triglyceride, verwendet oder wässrige Injektionssuspensionen, welche viskositätserhöhende Stoffe, z.B. Natriumcarboxymethylcellulose, Sorbit und/oder Dextran und gegebenenfalls auch Stabilisatoren enthalten.

Als topisch anwendbare pharmazeutische Präparate kommen in erster Linie Creme, Salben, Pasten, Schäume, Tinkturen und Lösungen, in Frage, die von etwa 0,5 bis etwa 20% des Wirkstoffs enthalten.

Creme sind Oel-in-Wasser Emulsionen, die mehr als 50% Wasser aufweisen. Als ölige Grundlage verwendet man in erster Linie Fettalkohole, z.B. Lauryl-, Cetyl- oder Stearylalkohol, Fettsäuren, z.B. Palmitin- oder Stearinsäure, flüssige bis feste Wachse, z.B. Isopropylmyristat, Wollwachs oder Bienenwachs, und/oder Kohlenwasserstoffe, z.B. Vaseline (Petrolatum) oder Paraffinöl. Als Emulgatoren kommen oberflächenaktive Substanzen mit vorwiegend hydrophilen Eigenschaften in Frage, wie entsprechende nichtionische Emulgatoren, z.B. Fettsäureester von Polyalkoholen oder Ethylenoxidaddukte davon, wie Polyglycerinfettsäureester oder Polyoxyethylensorbitanfettsäureester (Tweens), ferner Polyoxyethylenfettalkoholether oderfettsäureester, oder entsprechende ionische Emulgatoren, wie Alkalimetallsalze von Fettalkoholsulfaten, z.B. Natriumlaurylsulfat, Natriumcetylsulfat oder Natriumstearylsulfat, die man üblicherweise in Gegenwart von Fettalkoholen, z.B. Cetylalkohol oder Stearylalkohol, verwendet. Zusätze zur Wasserphase sind u.a. Mittel, welche die Austrocknung der Creme verhindern, z.B. Polyalkohole, wie Glycerin, Sorbit, Propylenglykol und/oder Polyethylenglykole, ferner Konservierungsmittel, Riechstoffe etc.

Salben sind Wasser-in-Oel-Emulsionen, die bis zu 70%, vorzugsweise jedoch von etwa 20% bis etwa 50% Wasser oder wässrige Phasen enthalten' Als Fettphase kommen in erster Linie

Kohlenwasserstoffe, z.B. Vaseline, Paraffinöl und/oder Hartparaffine in Frage, die zur Verbesserung des Wasserbindungsvermögens vorzugsweise geeignete Hydroxyverbindungen, wie Fettalkohole oder Ester davon, z.B. Cetylalkohol oder Wollwachsalkohole, bzw. Wollwachs enthalten. Emulgatoren sind entsprechende lipophile Substanzen, wie Sorbitanfettsäureester (Spans), z.B. Sorbitanoleat und/oder Sorbitanisostearat. Zusätze zur Wasserphase sind u.a. Feuchthaltungsmittel, wie Polyalkohole, z.B. Glycerin, Propylenglykol, Sorbit und/oder Polyethylenglykol, sowie Konservierungsmittel, Riechstoffe etc.

Fettsalben sind wasserfrei und enthalten als Grundlage insbesondere Kohlenwasserstoff, z.B. Paraffin, Caseline und/oder flüssige Paraffine, ferner natürliches oder partialsynthetisches Fett, z.B. Kokosfettsäuretriglycerid, oder vorzugsweise gehärtete Oele, z.B. hydriertes Erdnussoder Rizinusöl, ferner Fettsäurepartialester des Glycerins, z.B. Glycerinmono- und distearat, sowie z.B. die im Zusammenhang mit den Salben erwähnten, die Wasseraufnahmefähigkeit steigernden Fettalkohole, Emulgatoren und/oder Zusätze.

Pasten sind Creme und Salben mit sekretabsorbierenden Puderbestandteilen, wie Metalloxiden, z.B. Titanoxid oder Zinkoxid, ferner Talk und/oder Aluminiumsilikate, welche die Aufgabe haben, vorhandene Feuchtigkeit oder Sekrete zu binden.

Schäume werden aus Druckbehältern verabreicht und sind in Aerosolform vorliegende flüssige Oel-in-Wasser-Emulsionen, wobei halogenierte Kohlenwasserstoffe, wie Chlorfluorniederalkane, z.B. Dichlordifluormethan und Dichlortetrafluorethan, als Treibmittel verwendet werden. Als Oelphase verwendet man u.a. Kohlenwasserstoffe, z.B. Paraffinol, Fettalkohole, z.B. Cetylalkohol, Fettsäureester, z.B. Isopropylmyristat, und/oder andere Wachse. Als Emulgatoren verwendet man u.a. Gemische von solchen mit vorwiegend hydrophilen Eigenschaften, wie Polyoxyethylen-sorbitanfettsäureester (Tweens), und solchen mit vorwiegend lipophilen Eigenschaften, wie Sorbutanfettsäureester (Spans). Dazu kommen die üblichen Zusätze, wie Konservierungsmittel, etc.

Tinkturen und Lösungen weisen meistens eine wässrig-äthanolische Grundlage auf, der u.a. Polyalkohole, z.B. Glycerin, Glykole, und/oder Polyethylenglykol, als Feuchthaltemittel zur Herabsatzung der Verdunstung und rückfettende Substanzen, wie Fettsäureester mit niedrigen Polyethylenglykolen, d.h. im wässrigen Gemisch lösliche, lipophile Substanzen als Ersatz für die der Haut mit dem Aethanol entzogenen Fettsubstanzen, und, falls notwendig, andere Hilfs- und Zusatzmittel beigegeben sind.

Die Herstellung der topisch verwendbaren pharmazeutischen Präparate erfolgt in an sich bekannter Weise, z.B. durch Lösen oder Suspendieren des Wirkstoffs in der Grundlage oder in einem Teil davon, falls notwendig. Bei Verarbeitung des Wirkstoffs als Lösung wird dieser in der Regel von der Emulgierung in einer der beiden Phasen gelöst; bei Verarbeitung als Suspension wird er nach der Emulgierung mit einem Teil der Grundlage vermischt und dann dem Rest der Formulierung beigegeben.

Die vorliegende Erfindung betrifft ebenfalls die Verwendung der Verbindungen der Formeln I und der Salze von solchen Verbindungen mit salzbildenden Eigenschaften, vorzugsweise zur Behandlung von Entzündungen, in erster Linie von entzündlichen Erkrankungen des rheumatischen Formenkreises, besonders der chronischen Arthritis.

De nachfolgenden Beispiele illustrieren die oben beschriebene Erfindung; sie sollen jedoch diese in ihrem Umfang in keiner Weise einschränken. Temperaturen sind in Celsiusgraden angegeben.

Die den Verbindungen der Formel I zugrundeliegende Bezeichnung der Verknüpfungsstellen im Pyrimido-Indol-Rungsystem

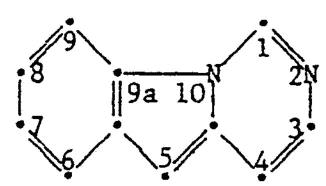

ist in der Literatur uneinheitlich'

So werden die älteren Literaturstellen die Verknüpfungstellen im Ringsystem mit [3,4-a] bezeichnet während neuerdings die Bezeichnung [1,6-a] verwendet wird.

Aus prinzipiellen Gründen wird nachfolgend für das vorstehend angegebene Ringgerüst folgende Nomenklatur vorgezogen:

**Pyrimido[1,6-a]indol**

**Beispiel 1:**

38 g 7-Flour-1-phenyl-3,4-dihydro-pyrimidol[1,6-a] indol-5-essigsäureethylester werden in 200 ml Diphenyläther in Gegenwart von 10 g Palladiumkohle (10%ig) unter Rühren 50 Minuten auf 260° erhitzt. Man zieht den Diphenylether unter vermindertem Druck ab, nimmt den Eindampfrückstand in Diethylether auf, filtriert vom Katalysator ab und engt bis zur beginnenden Kristallisation ein. Dann verrührt man mit Hexan und wenig Diethylether und saugt ab. Man erhält den 7-Fluor-1-phenyl-pyrimido[1,6-a]indol-5-essigsäureethylester vom Smp. 91-93°'

Das Ausgangsmaterial kann z.B. wie folgt hergestellt werden:

a) 131 g (0,45 Mol) 3-Benzyl-6-fluor-tetrahydro-γ-carbolin werden in 1300 ml Acetonitril bei 40°

unter Rühren gelöst und innerhalb von 10 Minuten 94 g (0,55 Mol) Benzylbromid zugesetzt. Nach kurzer Zeit beginnt das Benzylammoniumderivat auszukristallisieren. Es wird noch ca. 15 Stunden bei Raumtemperatur weitergerührt, danach im Eisbad gekühlt und vom Kristallisat abgesaugt.

b) 464 g (1 Mol) des so hergestellten N,N-Dibenzyl-6-fluor-tetrahydro-γ-carbolininiumbromids werden in 4250 ml Methancl unter Erwärmen auf 65° gelöst und innerhaln von 5 Minuten eine Lösung von 196 g (4 Mol) Natriumcyanid in 500 ml Wasser unter Rühren zugesetzt. Die Lösung wird 3 Stunden unter Rückfluss zum Sieden erhitzt. Beim Abkühlen kristallisiert nach Animpfen 2-(Dibenzylamino-ethyl)-3-cyanomethyl-5-fluor-indol in farblosen Kristallen.

c) 220 g (0,537 Mol) des nach b) hergestellten Nitrils werden in 300 ml abs. Ethanol gelöst und die Lösung bei −5° mit trockenem Chlorwasserstoff gesättigt. Anschliessend wird 5 1/2 Tage bei 20° gerührt. Man lässt die ausgeschiedenen Kristalle absitzen, dekantiert von der überstehenden Lösung ab, löst den Bodensatz in 2000 ml Eiswasser und rührt etwa 3 Stunden bei 20°. Dann wird unter Eiskühlung mit konz. Ammoniaklösung alkalisch gestellt und mit 1500 ml Toluol Eiswasser ausgerührt. Die abgetrennte Toluolphase wird mit Wasser gewaschen, über Natriumsulfat getrocknet und über 1000g Aluminiumoxid (Akt. Stufe 3) filtriert und mit Toluol nachgewaschen. Nach Abdestillieren des Toluols verbleibt ein hellbraunes Oel, das ohne weitere Reinigung der Hydrierung nach d) unterworfen wird.

d) 181,3 g des nach c) erhaltenen 2-Dibenzylamino-5-fluor-indol-3-essigsäureethylesters werden in 1500 ml abs. Alkohol gelöst, und unter Zusatz von 18 g Pd/C (5%) bei Normaldruck und 20-35° hydriert. Nach Aufnahme von 12 500 ml $H_2$ werden nochmals 18 g Katalysator zugesetzt und bis zum Stillstand der $H_2$-Aufnahme von insgesamt 17 300 ml weiterhydriert. Nach Abfiltrieren vom Katalysator und Nachwaschen mit Methylenchlorid wird die Lösung zur Trockne eingedampft und der Rückstand in 250 ml Ether gelöst. Nach Animpfen kristallisiert der 2-Amino-ethyl-5-fluor-indol-3-essigsäureethylester in farblosen Kristallen.

e) 61,7 g (0,223 Mol) des nach d) hergestellten 2-Aminoethyl-5-fluor-indol-3-essigsäureethylesters werden in 600 ml Methylenchlorid gelöst und die Lösung mit 150 ml 2N-Natronlauge überschichtet. Unter starkem Rühren wird bei 0-5° innerhalb 2 1/2 Stunden eine Lösung von 43 g (0,245 Mol) Benzoylchlorid zugesetzt und danach noch 1 Stunde ausgerührt. Die Methylenchloridphase wird dann abgetrennt, mit Wasser gewaschen, über $MgSO_4$ getrocknet und zur Trockne eingedampft. Der Rückstand kristallisiert beim Aufnehmen in Ether. Man erhält den 2-(2-Benzoyl-aminoethyl)-5-fluor-indol-3-essigsäureethylester.

f) 37 g (0,154 Mol) 2-(2-Benzoylamino-ethyl)-5-fluor-indol-3-essigsäure-ethylester werden in 160 ml Phosphoroxychlorid 3 Stunden unter Rückfluss zum Sieden erhitzt. Das überschüssige Phosphoroxychlorid wird dann bei 60° im Vakuum abdestilliert und der Rückstand mit 650 ml Eiswasser verrührt. Die wässrige Lösung wird durch Filtrieren über Diatomeenerde geklärt, mit konzentrierter Ammoniaklösung alkalisch gemacht und mit 400 ml Ether extrahiert. Die Etherphase wird über Natriumsulfat getrocknet und zur Trockne eingeengt. Den Rückstand löst man in 150 ml Aceton und versetzt mit 16 ml einer etwa 4-normalen Chlorwasserstoff-Lösung in Ether, Nach Animpfen kristallisiert das Hydrochlorid des 7-Fluor-1-phenyl-3,4-dihydro-pyrimido[1,6-a]indol-5-essigsäure-ethylesters in gelblichen Kristallen,die aus 2N-Salzsäure umkristallisiert werden können. Die aus dem Salz freigesetzte Base schmilzt nach Umkristallisieren aus Ether bei 56-58°.

**Beispiel 2:**

In analoger Weise wie in Beispiel 1 beschrieben erhält man ausgehend von 2-Aminoethyl-5-fluor-indol-3-essigsäureethylester und p-Methansulfinylbenzoylchlorid über den 7-Fluor-1-(p-methansul-finylphenyl)-3,4-dihydro-pyrimido[1,6-a]indol-5-essigsäureethylester (Smp. des Hydrochlorids 208-210°) den 7-Fluor-1-(p-methansulfinyl-phenyl)-pyrimido[1,6-a]indol-5-essigsäureethylester vom Smp. 133-135°.

**Beispiel 3:**

In analoger Weise wie in Beispiel 1 beschrieben erhält man ausgehend von 2-Aminoethyl-indol-3-essigsäureethylester und 2-The-noylchlorid über den 1-(2-Thienyl)-3,4-dihydro-pyrimido[1,6-a]indol-5-essigsäureethylester (Smp. des Hydrochlorids 153-157°) den 1-(2-Thie-nyl)-pyrimido[1,6-a]indol-5-essigsäureethylester vom Smp. 94-95°'

**Beispiel 4:**

In analoger Weise wie in Beispiel 1 beschrieben erhält man ausgehend von 2-Aminoethyl-5-methoxy-indol-3-essigsäureethylester und 2-Picolylchlorid über den 7-Methoxy-1-(2-picolinyl)-3,4-dihydro-pyrimido[1,6-a]indol-5-essigsäureethylester den 7-Methoxy-1-(2-picolinyl)-pyrimido[1,6-a]indol-5-essigsäureethylester vom Smp. 103-104°.

**Beispiel 5:**

12,2 g 1-Phenyl-pyrimido[1,6-a]indol-5-essigsäureethylester (Smp. 59-62°), hergestellt aus 2-Aminoethyl-indol-3-essigsäure-ethylester und Benzoylchlorid über den 1-Phenyl-3,4-dihyfro-pyrimido [1,6-a]indol-5-essigsäureethylester (Smp. 83-84°), werden mit 30 ml Ethanol und 40 ml 2N-Natronlauge 3 Stunden bei Raumtemperatur gerührt. Man säuert mit konzentrierter Salzsäure auf pH = 3 an und versetzt unter Rühren mit 30 ml Propylenoxid. Nach kurzer Zeit kristallisiert die 1-Phenyl-pyrimido[1,6-a]indol-5-essigsäure aus. Sie wird abfiltriert und aus 90%igem Ethanol umkristallisiert. Smp. 198-204° (Zers.).

**Beispiel 6:**

In analoger Weise wie in Beispiel 5 beschrieben erhält man ausgehend von 7-Fluor-1-(p-methansulfinylphenyl)-pyrimido[1,6-a] indol-5-essigsäureethylester die 7-Fluor-(p-methansulfinylphenyl)-pyrimido[1,6-a]indol-5-essigsäure vom Smp. 218-224° (Zers.).

**Beispiel 7:**

In analoger Weise wie in Beispiel 5 beschrieben erhält man ausgehend von 7-Fluor-1-phenyl-pyrimido[1,6-a]indol-5-essigsäureethylester die 7-Fluor-1-phenyl-pyrimido[1,6-a]indol-5-essigsäure, Smp. 217-220°.

**Beispiel 8:**

In analoger Weise wie in Beispiel 5 beschrieben erhält man ausgehend von 1-(2-Thienyl)-pyrimido[1,6-a]indol-5-essigsäureethylester die 1-(2-Thienyl)-pyrimido[1,6-a]indol-5-essigsäure, Smp. 196-200° (Zers.).

**Beispiel 9:**

In analoger Weise wie in Beispiel 5 beschrieben erhält man ausgehend von 7-Methoxy-1-(2-picolinyl)-pyrimido[1,6-a]indol-5-essigsäureethylester die 7-Methoxy-1-(2-picolinyl)-pyrimido[1,6-a]indol-5-essigsäure, Smp. 201-206°.

**Beispiel 10:**

In analoger Weise wie in den Beispielen 1 und 5 beschrieben, erhält man ferner

7-Fluor-1-(p-methoxyphenyl)-pyrimido[1,6-a]indol-5-essigsäure, Smp. 220-225°, und deren Ethylester,

7-Methoxy-1-(p-fluorphenyl)-pyrimido[1,6-a]indol-5-essigsäure und deren Ethylester, sowie

7-Fluor-1-(2-thienyl)-pyrimido[1,6-a]indol-5-essigsäure, Smp.233-236°,

7-Fluor-1-(2-thienyl)-pyrimido[1,6-a]indol-5-essigsäureethylester.

**Beispiel 11:**

3 g 7-Fluor-1-phenyl-3,4-dihydro-pyrimido[1,6-a]indol-5-essigsäureethylester werden mit 1 g Selendioxid in 50 ml Chlorbenzol dampft zur Trockne ein nimmt den Rückstand in 20 ml Essigsäureester auf, filtriert vom Selen ab, wäscht die Lösung mit Natriumbikarbonatlösung neutral und dampft zur Trockne ein. Der Rückstand wird in Ether aufgenommen und über 80 g Kieselgel chromatographiert. Der 7-Fluor-1-phenyl-pyrimido[1,6-a]indol-5-essigsäureethylester wird mit Hexan/ Essigester (4:1) eluiert und kristallisiert aus Ether/Hexan, Smp. 91-93°.

**Beispiel 12:**

Zu einer Lösung von 3,38 g 1-(2-Thienyl)-3,4-dihydro-pyrimido[1,6-a]indol-5-essigsäureethylester in 20 ml Dimethoxyethan tropft man innerhalb von 0,5 Stunden eine Lösung von 0,01 Mol Diphenylselenium-bis-(trifluoracetat) (hergestellt nach J. Amer. Chem. Soc., 103, 4642 (1981)). Man lässt noch 3 Stunden bei Raumtemperatur stehen, 4' dampft zur Trockne ein, nimmt den Rückstand in Ether auf, wäscht mit Natriumbikarbonatlösung und chromatographiert über 80 g Kieselgel. Der 1-(2-Thienyl)-pyrimido[1,6-a]indol-5-essigsäureethylester wird mit Hexan/Essigester (4:1) eluiert und kristallisiert aus Ether/Hexan in gelben Kristallen, Smp. 94-95°'

**Beispiel 13:**

Tabletten, enthaltend 25 mg Wirkstoff, z.B. 7-Fluor-1-phenyl-pyrimido[(1,6-a]indol-5-essigsäure oder ein Salz, z.B. das Hydrochlorid davon, können folgendermassen hergestellt werden:

**Bestandteile** (für 1000 Tabletten)

| | |
|---|---|
| Wirkstoff | 25,0 g |
| Lactose | 100,7 g |
| Weizstärke | 7,5 g |
| Polyethylenglykol 6000 | 5,0 g |
| Talkum | 5,0 g |

| | |
|---|---|
| Magnesiumstearat | 1,8 g |
| entmineralisiertes Wasser | q.s. |

**Herstellung:**

Sämtliche festen Ingredienzien werden zunächst durch ein Sieb mit 0,6 mm Maschenweite getrieben. Dann wird der Wirkstoff, die Lactose, das Talkum, das Magnesiumstearat und die Hälfte der Stärke vermischt. Die andere Hälfte der Stärke wird in 40 ml Wasser suspendiert und diese Suspension zu einer siedenden Lösung des Polyethylenglykols in 100 ml Wasser hinzugegeben. Der erhaltene Stärkekleister wird zu der Hauptmenge hinzugegeben und das Gemisch, wenn nötig unter Hinzufügen von Wasser, granuliert. Das Granulat wird über Nacht bei 35° etrocknet, durch ein Sieb mit 1,2 mm Maschenweite getrieben und zu beidseitig konkaven Tabletten von etwa 6mm Durchmesser verpresst.

In analoger Weise können auch Tabletten, enthaltend jeweils 25 mg einer anderen der in den Beispielen 1 bis 12 genannten Verbindungen der Formel I hergestellt werden, wobei die Verbindungen auch in Form von Säureadditionssalzen, wie Hydrochloriden, und Verbindungen, worin $R_2$ 1-Carboxymethyl ist, auch in Form von Salzen und Basen, wie Natrium-, Kalium- oder Zinksalzen vorliegen können.

**Beispiel 14:**

Kautabletten, enthaltend 30 mg Wirkstoff, z.B. 7-Fluor-1-phenyl-pyrimido[1,6-a]indol-5-essigsäure oder ein Salz, z.B. das Hydrochlorid davon, können z.B. folgendermassen hergestellt werden:

**Zusammensetzung** (für 1000 Tabletten)

| | |
|---|---|
| Wirkstoff | 30,0 g |
| Mannit | 267,0 g |
| Lactose | 179,5 g |
| Talkum | 20,0 g |
| Glycin | 12,5 g |
| Stearinsäure | 10,0 g |
| Saccharin | 1,0 g |
| 5%ige Gelatinelösung q.s. | |

**Herstellung:**

Alle festen Ingredienzien werden zunächst durch ein Sieb mit 0,25 mm Maschenbreite getrieben. Der Mannit und die Lactose werden gemischt, unter Hinzufügen von Gelatinelösung granuliert durch ein Sieb mit 2 mm Maschenweite getrieben, bei 50° getrocknet und nochmals durch ein Sieb mit 1,7 mm Maschenweite getrieben. Der Wirkstoff, das Glycin und das Saccharin werden sorgfältig vermischt, der Mannit, das Lactosegranulat, die Stearinsäure und das Talkum hinzugegeben, das Ganze gründlich vermischt und zu beidseitig konkaven Tabletten von etwa 10 mm Durchmesser mit Bruchrille auf der Oberseite verpresst. In analoger Weise können auch Kautabletten enthaltend jeweils 30 mg einer anderen der in den Beispielen 1 bis 12 genannten Verbindungen der Formel I hergestellt werden, wobei die Verbindungen auch in Form von Säureadditionssalzen, wie Hydrochloriden, und Verbindungen, worin $R_2$ 1-Carboxymethyl ist, auch in Form von Salzen mit Basen, wie Natrium-, Kalium oder Zinksalzen vorliegen können.

**Beispiel 15:**

Tabletten enthaltend 100 mg Wirkstoff, z.B. 7-Fluor-1-phenyl-pyrimido[1,6-a]indol-5-essigsäureethylester können folgendermassen hergestellt werden:

**Zusammensetzung** (für 1000 Tabletten)

| | |
|---|---|
| Wirkstoff | 100,0 g |
| Lactose | 248,5 g |
| Maisstärke | 17,5 g |
| Polyethylenglykol 6000 | 5,0 g |
| Talkum | 15,0 g |
| Magnesiumstearat | 4,0 g |
| entmineralisiertes Wasser q.s. | |

**Herstellung:**

Die festen Ingredienzien werden zunächst durch ein Sieb mit 0,6 mm Maschenweite getrieben. Dann werden Wirkstoff, Lactose, Talkum, Magnesium-stearat und die Hälfte der Stärke innig vermischt. Die andere Hälfte der Stärke wird in 65 ml Wasser suspendiert und diese Suspension zu einer siedenden Lösung des Polyethylenglykols in 260 ml Wasser hinzugegeben. Der erhaltene Kleister wird zu den pulverförmigen Substanzen hinzugefügt, das Ganze vermischt und granuliert, erforderlichenfalls unter Zugabe von Wasser. Das Granulat wird über Nacht bei 35° getrocknet, durch ein Sieb mit 1,2 mm Maschenweite getrieben und zu beidseitig konkaven Tabletten von etwa 10 mm Durchmesser mit Bruchkerbe auf der Oberseite verpresst.

In analoger Weise können auch Tabletten, enthaltend 100 mg einer anderen Verbindung der Formel I gemäss den Beispielen 1 bis 12 hergestellt werden, wobei Verbindungen auch in

Form von Säureadditionssalzen, wie Hydrochloriden, und Verbindungen, worin $R_2$ 1-Carboxymethyl ist, auch in Form von Salzen mit Basen, wie Natrium-, Kalium- oder Zinksalzen, vorliegen können.

**Patentansprüche** für die Verstragsstaaten BE CH DE FR IT LI LU NL SE

1. N,N'-Überückte Carbonsäureamidine der allgemeinen Formel

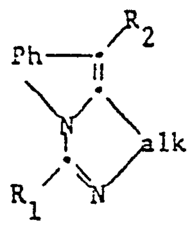

(I),

worin $R_1$ gegebenenfalls in p-Stellung durch Niederalkylthio mit bis und mit 4 C-Atomen, durch Niederalkansulfinyl mit bis und mit 4 C-Atomen, Niederalkoxy mit bis und mit 4 C-Atomen oder Halogen der Atomnummer bis und mit 35 substituiertes Phenyl, Thienyl oder gegebenenfalls durch Niederalkyl mit bis und mit 4 C-Atomen substituiertes Pyridyl bedeutet, $R_2$ Carboxymethyl oder Niederalkoxycarbonylmethyl mit bis und mit 5 C-Atomen ist, Ph gegebenenfalls in p-Stellung zum Stickstoffatom durch Niederalkoxy mit bis und mit 4 C-Atomen oder Halogen der Atomnummer bis und mit 35 substituiertes 1,2-Phenylen darstellt und alk Vinylen bedeutet,und ihre Salze, mit der Massgabe, dass, wenn $R_1$ p-Methylthiophenyl ist und Ph in p-Stellung zum N-Atom durch Fluor substituiertes 1,2-Phenylen bedeutet, $R_2$ von Carboxymethyl oder Ethoxycarbonylmethyl verschieden ist, mit der weiteren Massgabe dass venn $R_1$ Phenyl ist und ph unsubstituiertes 1,2-phenylen bedeutet oder wenn $R_1$ p-Chlorphenyl ist und Ph in p-Stellung zum Stickstoffatom durch Methoxv substituiertes 1,2-Phenylen darstellt, $R_2$ jeweils von Ethoxycarbonylmethyl verschieden ist.

2. Verbindungen der Formel I gemäss Anspruch 1 worin $R_1$ unsubstituiertes phenvl p-Methoxyphenyl oder p-Methansulfinylphenyl bedeutet, $R_2$ jewcils Ethoxycarbonylmethyl ist, Ph jeweils in p-Stellung zum N-Atom durch Fluor substituiertes 1,2-Phenylen darstellt und alk jeweils Winylen ist, worin $R_1$ unsubstituiertes 2 Thienyl, unsubstituiertes Phenyl, p-Methansulfinylphenyl oder p-Methoxyphenyl bedeutet, $R_2$ jeweils Carboxymethyl ist, Ph jeweils in p-Stellung zum N-Atom durch Fluor substituiertes 1,2-Phenylen darstellt und alk jeweils Vinylen ist, worin $R_1$ jeweils unsubstituiertes 2-Thienyl bedeutet, $R_2$ Carboxymethyl oder Ethoxycarbonylmethyl ist, Ph jeweils unsubstituiertes 1,2-Phenylen darstellt und alk jeweils Vinylen ist, worin $R_1$ unsubstituiertes Phenyl bedeutet, $R_2$ Carboxymethyl ist, Ph unsubstituiertes 1,2-Phenylen darstellt und alk Vinylen ist, oder worin $R_1$ 2-Pi-colinyl oder p-Fluorphenyl bedeutet, $R_2$ jeweils Carboxymethyl oder Ethoxycarbonylmethyl ist, Ph jeweils in p-Stellung zum N-Atom durch Methoxy substituiertes 1,2-Phenylen darstellt und alk jeweils Vinylen ist, und ihre Salze.

3. Verbindungen der Formel I gemäss Anspruch 1, worin Ph unsubstituiertes 1,2-Phenylen bedeutet und $R_1$ Phenyl und $R_2$ Carboxymethyl oder $R_1$ 2-Thienyl und $R_2$ Carboxy- oder Ethoxycarbonylmethyl darstellt, oder Ph in p-Stellung zum N-Atom durch Fluor substituiertes 1,2-Phenylen bedeutet und $R_1$ Phenyl, p-Methansulfinylphenyl oder p-Methoxyphenyl oder $R_2$ Carboxy- oder Ethoxycarbonylmethyl darstellt, oder Ph in p-Stellung zum N-Atom durch Methoxy substituiertes 1,2-Phenylen bedeutet und $R_1$ p-Fluorphenyl oder 2-Picolinyl und $R_2$ Carboxy- oder Ethoxycarbonylmethyl darstellt, wobei alk jeweils Vinylen bedeutet, und ihre Salze.

4. 7-Fluor-1-phenyl-pyrimido[1,6-a]indol-5-essigsäureethylester oder ein Salz davon.

5. 7-Fluor-1-(2-thienyl)-pyrimido[1,6-a]indol-5-essigsäureethylester oder ein Salz davon.

6. 1-(2-Thienyl)-pyrimido([,6-a]indol-5-essigsäureethylester oder ein Salz davon.

7. 7-Methoxy-1-(2-picolinyl)-pyrimido[1,6-a]-indol-5-essigsäureethylester oder ein Salz davon.

8. 1-Phenyl-pyrimido[1,6-a]indol-5-essigsäure oder ein Salz davon.

9. 7-Fluor-1-(p-methansulfinylphenyl)-pyrimido[1,6-a]indol-5-essigsäureethylester oder ein Salz davon.

10. 7-Fluor-1-(p-methansulfinylphenyl)-pyrimido[1,6-a]indol-5-essigsäure oder ein Salz davon.

11. 7-Fluor-1-phenyl-pyrimido[1,6-a]indol-5-essigsäure oder ein Salz davon.

12. 1-(2-Thienyl)-pyrimido[1,6-a]indol-5-essigsäure oder ein Salz davon.

13 7-Methoxy-1-(2-picolinyl)-pyrimido[1,6-a]indol-5-essigsäure oder ein Salz davon.

14. 7-Fluor-1-(p-methoxyphenyl)-pyrimido[1,6-a]indol-5-essigsäure oder ein Salz davon.

15. 7-Fluor-1-(p-methoxyphenyl)-pyrimido[1,6-a]indol-5-essigsäureethylester oder ein Salz davon.

16. 7-Methoxy-1-(p-fluorphenyl)-pyrimido[1,6-a]indol-5-essigsäure oder ein Salz davon.

17. 7-Methoxy-1-(p-fluorphenyl)-pyrimido[1,6-a]indol-5-essigsäureethylester oder ein Salz davon.

18. 7-Fluor-1-(2-thienyl)-pyrimido[1,6-a]indol-5-essigsäure oder ein Salz davon.

19. Verbindungen der Formel I gemäss einem der Ansprüche 1-18 mit antiphlogistischer und/oder analgetischer Wirkung.

20. Pharmazeutische Präparate enthaltend mindestens eine Verbindung gemäss einem der Ansprüche 1-19 in freier Form oder in Form eines pharmazeutisch verwendbaren Salzes zusammen mit den üblichen pharmazeutischen Hilfs- und

Trägerstoffen.

21. Verwendung von Verbindungen der Formel I gemäss einem der Ansprüche 1-19 als Arzneimittel.

22. Verwendung von Verbindungen der Formel I gemäss einem der Ansprüche 1-19 bei der Behandlung von Schmerzzuständen oder entzündlicher Prozesse.

23. Verwendung von Verbindungen der Formel I gemäss einem der Ansprüche 1-19 zur Herstellung von pharmazeutischen Präparaten.

24. Verbindung der Formel I gemäss einem der Ansprüche 1-19 zur Anwendung in einem Verfahren zur therapeutischen Behandlung des menschlichen oder tierischen Körpers.

25. Verfahren zur Herstellung von Verbindungen der allgemeinen Formel (I) gemäss einem der Ansprüche 1-19 und ihrer Salze, dadurch gekennzeichnet, dass man aus Verbindungen der allgemeinen Formel

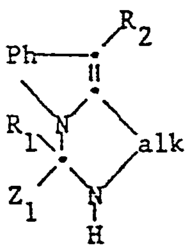

(II),

worin $Z_1$ gegebenenfalls funktionell abgewandeltes Hydroxy oder die Mercaptogruppe bedeutet, oder Salzen davon unter Einführung einer zusätzlichen Bindung H-$Z_1$ abspaltet oder Verbindungen der Formel

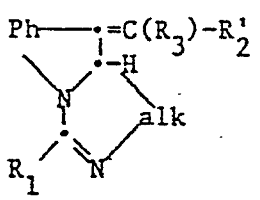

(V),

worin $R'_2$ gegebenenfalls wie unter $R_2$ angegeben verestertes Carboxy und $R_3$ Wasserstoff bedeutet, oder Salze davon isomerisiert oder eine Verbindung der allgemeinen Formel

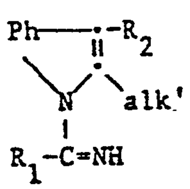

(VIII),

worin alk' eine Gruppe der Formel $-CH=CH-Z_6$ oder $-CH_2-CH=Z'_6$ ist $Z_6$ gegebenenfalls funktionell abgewandeltes Hydroxy oder Amino bzw. $Z'_6$ Oxo oder Imino bedeutet, bzw. ein Salz davon cyclisiert oder in einer Verbindung der Formel

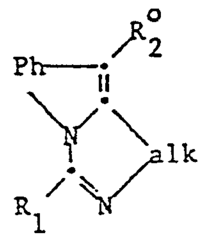

(X), .

worin $R^o_2$ einen in die Gruppe $R_2$ überfürbaren Rest bedeutet, oder Salzen davon $R^o_2$ solvolytisch oder oxidativ in die Gruppe $R_2$ überführt oder Verbindungen der Formel

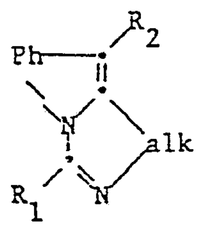

(I),

worin alk Ethylen bedeutet unter Abspaltung von Wasserstoff bei gleichzeitiger Bildung einer zusätzlichen Bindung dehydriert und gewünschtenfalls eine erfindungsgemäss erhältliche Verbindung in eine andere Verbindung der Formel I bzw. eine erfindungsgemäss erhältliche freie Verbindung der Formel I in ein Salz oder ein verfahrensgemäss erhältliches Salz in die freie Verbindung der Formel I bzw. in ein anderes Salz überführt.

26. Verfahren nach Anspruch 25, dadurch gekennzeichnet, dass man in einer Verbindung der Formel (X), worin $R^o_2$ eine Gruppe der Formel $-CH(R_3)-R''_2$ bedeutet, in der $R_3$ Wasserstoff ist und $R''_2$ von $R'_2$ verschiedenes funktionell abgewandeltes Carboxy bedeutet und $R'_2$ mit dem Carboxy- bzw. Niederalkoxycarbonyl-Teil von $R_2$ identisch ist, $R^o_2$ durch Hydrolyse oder Alkoholyse in $R_2$ überführt oder eine Verbindung der Formel (I), worin alk Ethylen bedeutet, unter Abspaltung von Wasserstoff in Gegenwart eines Dehydrierungsmittels bei gleichzeitiger Bildung einer zusätzlichen Doppelbindung dehydriert.

27. Verfahren gemäss Anspruch 25 oder 26, dadurch gekennzeichnet, dass man Verbindungen der Formel

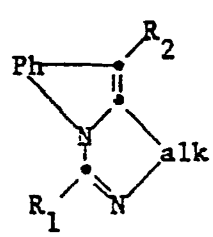

(I),

worin alk Ethylen bedeutet, unter Abspaltung von Wasserstoff bei gleichzeitiger Bildung einer zusätzlichen Bindung dehydriert und gewünschtenfalls in einer erfindungsgemäss

erhältlichen Verbindung der Formel I, worin Ph, alk und $R_1$ die angegebenen Bedeutungen haben und $R_2$ Niederalkoxycarbonyl-methyl bedeutet, Niederalkoxycarbonyl-methyl $R_2$ zu Carboxymethyl $R_2$ hydrolysiert.

28. Verfahren zur Herstellung pharmazeutischer Präparate, dadurch gekennzeichnct, dass man eine Verbindung gemäss einem der Ansprüche 1-19, in freier Form oder in Form eines pharmazeutisch verwendbaren Salzes, mit üblichen pharmazeutischen Hilfs- bzw. Trägerstoffen vermischt.

**Patentansprüche** für den Vertragsstaat AT

1. Verfahren zur Herstellung von Verbindungen der allgemeinen Formel

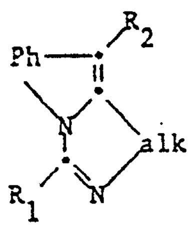

(I),

worin $R_1$ gegebenenfalls in p-Stellung durch Niederalkylthio mit bis und mit 4 C-Atomen durch Niederalkansulfinyl mit bis und mit 4 C-Atomen, Niederalkoxy mit bis und mit 4 C-Atomen oder Halogen der Atomnummer bis und mit 35 substituiertes Phenyl, Thienyl oder gegebenenfalls durch Niederalkyl mit bis und mit 4 C-Atomen substituiertes Pyridyl bedeutet, $R_2$ Carboxymethyl oder Niederalkoxycarbonylmethyl mit bis und mit 5 C-Atomen ist, Ph gegebenenfalls in p-Stellung zum Stickstoffatom durch Niederalkoxy mit bis und mit 4 C-Atomen oder Halogen der Atomnummer bis und mit 35 substituiertes 1,2-Phenylen darstellt und alk Vinylen bedeutet, und ihre Salze, mit der Massgabe, dass, wenn $R_1$ p-Methylthiophenyl ist und Ph in p-Stellung zum N-Atom durch Fluor substituiertes 1,2-Phenylen bedeutet, $R_2$ von Carboxymethyl oder Ethoxycarbonylmethyl verschieden ist, mit der weiteren Massgabe, dass, wenn $R_1$ Phenyl ist und Ph unsubstituiertes 1,2-Phenylen bedeutet oder wenn $R_1$ p-Chlorphenyl ist und Ph in p-Stellung zum Stickstoffatom durch Methoxy substituiertes 1,2-Phenylen darstellt, $R_2$ jeweils von Ethoxycarbonylmethyl verschieden ist dadurch gekennzeichnet dass man aus Verbindungen der allgemeinen Formel

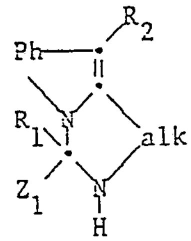

(II),

worin $Z_1$ gegebenenfalls funktionell abgewandeltes Hydroxy oder die Mercaptogruppe bedeutet, oder Salzen davon unter Einführung einer zusätzlichen Bindung $H-Z_1$ abspaltet oder Verbindungen der Formel

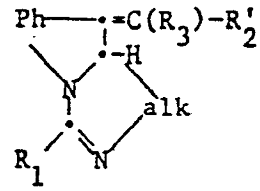

(V),

worin $R'_2$ gegebenenfalls wie unter $R_2$ angegeben verestertes Carboxy und $R_3$ Wasserstoff bedeutet oder Salze davon isomerisiert oder eine Verbindung der allgemeinen Formel

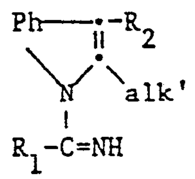

(VIII),

worin alk' eine Gruppe der Formel $-CH=CH-Z_6$ oder $-CH_2-CH=Z'_6$ ist $Z_6$ gegebenenfalls funktionell abgewandeltes Hydroxy oder Amino bzw $Z'_6$ Oxo oder Imino bedeutet, bzw. ein Salz davon cyclisiert oder in einer Verbindung der Formel

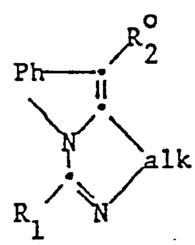

(X),

worin $R^o_2$ einen in die Gruppe $R_2$ überführbaren Rest bedeutet, oder Salzen davon $R^o_2$ solvolytisch oder oxidativ in die Gruppe $R_2$ überführt oder Verbindungen der Formel

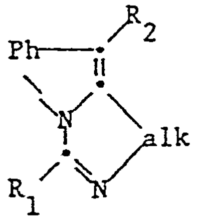

(I),

worin alk Ethylen bedeutet, unter Abspaltung von Wasserstoff bei gleichzeitiger Bildung einer zusätzlichen Bindung dehydriert und gewünschtenfalls eine erfindungsgemäss erhältliche Verbindung in eine andere Verbindung der Formel I bzw. eine erfindungsgemäss erhältliche freie Verbindung der Formel I in ein Salz oder ein verfahrensgemäss erhältliches Salz in die freie Verbindung der Formel I bzw. in ein anderes Salz überführt.

2. Verfahren nach Anspruch I, dadurch gekennzeichnet, dass man in einer Verbindung der Formel (X), worin $R^{o}_2$ eine Gruppe der Formel $-CH(R_3)-R''_2$ bedeutet in der $R_3$ Wasserstoff ist und $R''_2$ von $R'_2$ verschiedenes funktionell abgewandeltes Carboxy bedeutet und $R'_2$ mit dem Carboxy- bzw. Niederalkoxycarbonyl-Teil von $R_2$ identisch ist, $R^{o}_2$ durch Hydrolyse oder Alkoholyse in $R_2$ überführt oder eine Verbindung der Formel (I), worin alk Ethylen bedeutet, unter Abspaltung von Wasserstoff in Gegenwart eines Dehydrierungsmittels bei gleichzeitiger Bildung einer zusätzlichen Doppelbindung dehydriert.

3. Verfahren gemäss Ansprüchen 1 und 2, dadurch gekennzeichnet, dass man Verbindungen der Formel

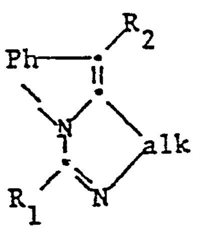

(I),

worin alk Ethylen bedeutet unter Abspaltung von Wasserstoff bei gleichzeitiger Bildung einer zusätzlichen Bindung dehydriert und gewünschtenfalls in einer erfindungsgemäss erhältlichen Verbindung der Formel I, worin Ph, alk und $R_1$ die angegebenen Bedeutungen haben und $R_2$ Niederalkoxycarbonyl-methyl bedeutet, Niederalkoxycarbonyl-methyl $R_2$ zu Carboxymethyl $R_2$ hydrolysiert.

4. Verfahren nach Anspruch 1 bis 3, zur Herstellung von Verbindungen der Formel I, worin $R_1$ unsubstituiertes Phenyl, p-Methoxyphenyl oder p-Methansulfinylphenyl bedeutet, $R_2$ jeweils Ethoxycarbonylmethyl ist, Ph jeweils in p-Stellung zum N-Atom durch Fluor substituiertes 1,2-Phenylen darstellt und alk jeweils Vinylen ist, worin $R_1$ unsubstituiertes 2-Thienyl, unsubstituiertes Phenyl, p-Methansulfinylphenyl oder p-Methoxyphenyl bedeutet, $R_2$ jeweils Carboxymethyl ist, Ph jeweils in p-Stellung zum N-Atom durch Fluor substituiertes 1,2-Phenylen darstellt und alk jeweils Vinylen ist, worin $R_1$ jeweils unsubstituiertes 2-Thienyl bedeutet, $R_2$ Carboxymethyl oder Ethoxycarbonylmethyl ist, Ph jeweils unsubstituiertes 1,2-Phenylen darstellt und alk jeweils Vinylen ist, worin $R_1$ unsubstituiertes Phenyl bedeutet, $R_2$ Carboxymethyl ist, Ph unsubstituiertes 1,2-Phenylen darstellt und alk Vinylen ist, oder worin $R_1$ 2-Picolinyl oder p-Fluorphenyl bedeutet, $R_2$ jeweils Carboxymethyl oder Ethoxycarbonylmethyl ist, Ph jeweils in p-Stellung zum N-Atom durch Methoxy substituiertes 1,2-Phenylen darstellt und alk jeweils Vinylen ist, und ihre Salze.

5. Verfahren nach Anspruch 1 bis 3, zur Herstellung von Verbindungen der Formel I, worin Ph unsubstituiertes 1,2-Phenylen bedeutet und $R_1$ Phenyl und $R_2$ Carboxymethyl oder $R_1$ 2-Thienyl und $R_2$ Carboxy- oderEthoxycarbonylmethyl darstellt, oder Ph in p-Stellung zum N-Atom durch Fluor substituiertes 1,2-Phenylen bedeutet und $R_1$ Phenyl, p-Methansulfinylphenyl oder p-Methoxyphenyl oder $R_2$ Carboxy- oder Ethoxycarbonylmethyl darstellt, oder P in p-Stellung zum N-Atom durch Methoxy substituiertes 1,2-Phenylen bedeutet und $R_1$ p-Fluorphenyl oder 2-Picolinyl und $R_2$ Carboxy- oder Ethoxycarbonylmethyl darstellt, wobei alk jeweils Vinylen bedeutet, und ihre Salze.

6. Verfahren nach Anspruch 1 bis 3. zur Herstellung von 7-Fluor-1-(2-thienyl)-pyrimido[1,6-a]indol-5-essigsäure oder einem Salz davon.

7. Verfahren nach Patentanspruch 1 bis 3. zur Herstellung von 7-Fluor-1-phenyl-pyrimido[1,6-a]indol-5-essigsaureethylester oder einem Salz davon.

8. Verfahren nach Anspruch 1 bis 3 zur Herstellung von 7-Fluor-1-(2-thienyl)-pyrimido[1,6-a]indol-5-essigsäureethylester oder einem Salz davon.

9. Verfahren nach Anspruch 1 bis 3 zur Herstellung von 1-(2-Thienyl)-pyrimido[1,6-a]indol-5-essigsäureethylester oder eirem Salz davon.

10. Verfahren nach Anspruch 1 bis 3 zur Herstellung von 7-Methoxy-1(2-picolinyl)-pyrimido[1,6-a]indol-5-essigsäureethylester oder einem Salz davon.

11. Verfahren nach Anspruch 1 bis 3 zur Herstellung von 1-Phenyl-pyrimido[1,6-a]indol-5-essigsäure oder einem Salz davon.

12. Verfahren nach Anspruch 1 bis 3 zur Herstellung von 7-Fluor-1-(p-methansulfinylphenyl)-pyrimido[1,6-a]indol-5-essigsäureethylester oder einem Salz davon.

13. Verfahren nach Anspruch 1 bis 3 zur Herstellung von 7-Fluor-1-(p-methansulfinylphenyl)-pyrimido [6-a]indol-5-essigsäure oder einem Salz davon.

14. Verfahren nach Anspruch 1 bis 3 zur Herstellung von 7-Fluor-1-phenyl-pyrimido[1,6-a]indol-5-essigsäure oder einem Salz davon.

15. Verfahren nach Anspruch 1 bis 3 zur

Herstellung von 1-(2-Thienyl)-pyrimido[1,6-a]indol-5-essigsäure oder einem Salz davon.

16. Verfahren nach Anspruch 1 bis 3 zur Herstellung von 7-Methoxy-1-(2-picolinyl)-pyrimido[1,6-a]indol-5-essigsäure oder einem Salz davon.

17. Verfahren nach Anspruch 1 bis 3 zur Herstellung von 7-Fluor-1-(p-methoxyphenyl)-pyrimido[1,6-a]indol-5-essigsäure oder einem Salz davon.

18. Verfahren nach Anspruch 1 bis 3 zur Herstellung von 7-Fluor-1-(p-methoxyphenyl)-pyrimido[1,6-a]indol-5-essigsäure oder einem Salz davon.

19. Verfahren nach Anspruch 1 bis 3 zur Herstellung von 7-Methoxy-1-(p-fluorphenyl)-pyrimido[1,6-a]indol-5-essigsäure oder einem Salz davon.

20. Verfahren nach Anspruch 1 bis 3 zur Herstellung von 7-Methoxy-1-(p-fluorphenyl)-pyrimido[1,6-a]indol-5-essigsäureethyl-ester oder einem Salz davon.

21. Verfahren zur Herstellung pharmazeutischer Präparate, dadurch gekennzeichnet dass man eine Verbindung gemäss einem der Ansprüche 1 und 4-20, in freier Form oder in Form eines pharmazeutisch verwendbaren Salzes, mit üblichen pharmazeutischen Hilfs- bzw. Trägerstoffen vermischt.

**Claims** for the Contracting States: DE, FR, CH, LI, IT, NL, BE, SE, LU

1. An N,N'-bridged carboxylic acid amidine of the general formula

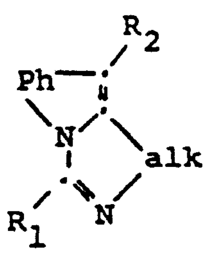 (I)

wherein $R_1$ is phenyl which is unsubstituted or substituted in the p-position by lower alkylthio containing up to and including 4 carbon atoms by lower alkanesulfinyl containing up to and including 4 carbon atoms by lower alkoxy containing up to and including 4 carbon atoms or by halogen having an atomic number of up to and including 35, or is thienyl, or pyridyl which is unsubstituted or substituted by lower alkyl containing up to and including 4 carbon atoms, $R_2$ is carboxymethyl or lower alkoxycarbonylmethyl containing up to and including 5 carbon atoms, Ph is 1,2-phenylene which is unsubstituted or substituted in the p-position to the nitrogen atom by lower alkoxy containing up to and including 4 carbon atoms or by halogen having an atomic number of up to and including 35, and alk is vinylene, or a salt thereof, with the proviso that if $R_1$ is p-methylthiophenyl and Ph is 1,2-phenylene substituted in the p-position to the nitrogen atom by fluorine, then $R_2$ is other than carboxymethyl or ethoxycarbonylmethyl, and with the further proviso that if $R_1$ is phenyl and Ph is unsubstituted 1,2-phenylene or if $R_1$ is p-chlorophenyl and Ph is 1,2-phenylene substituted in the p-position to the nitrogen atom by methoxy, then $R_2$ is other than ethoxycarbonylmethyl.

2. A compound of formula I according to claim 1, wherein $R_1$ is unsubstituted phenyl, p-methoxyphenyl or p-methanesulfinylphenyl, $R_2$ is ethoxycarbonylmethyl, Ph is 1,2-phenylene substituted in the p-position to the nitrogen atom by fluorine and alk is vinylene, or wherein $R_1$ is unsubstituted 2-thienyl, unsubstituted phenyl, p-methanesulfinylphenyl or p-methoxyphenyl, $R_2$ is carboxymethyl, Ph is 1,2-phenylene substituted in the p-position to the nitrogen atom by fluorine and alk is vinylene, or wherein $R_1$ is unsubstituted 2-thienyl, $R_2$ is carboxymethyl or ethoxycarbonylmethyl, Ph is unsubstituted 1,2-phenylene and alk is vinylene, or wherein $R_1$ is unsubstituted phenyl, $R_2$ is carboxymethyl, Ph is unsubstituted 1,2-phenylene and alk is vinylene, or wherein $R_1$ is 2-picolinyl or p-fluorophenyl, $R_2$ is carboxymethyl or ethoxycarbonylmethyl, Ph is 1,2-phenylene substituted in the p-position to the nitrogen atom by methoxy and alk is vinylene, or a salt thereof.

3. A compound of formula I according to claim 1, wherein Ph is unsubstituted 1,2-phenylene and $R_1$ is phenyl and $R_2$ is carboxymethyl or $R_1$ is 2-thienyl and $R_2$ is carboxymethyl or ethoxycarbonylmethyl, or Ph is 1,2-phenylene substituted in the p-position to the nitrogen atom by fluorine and $R_1$ is phenyl, p-methanesulfinylphenyl or p-methoxyphenyl and $R_2$ is carboxymethyl or ethoxycarbonylmethyl, or Ph is 1,2-phenylene substituted in the p-position to the nitrogen atom by methoxy and $R_1$ is p-fluorophenyl or 2-picolinyl and $R_2$ is carboxymethyl or ethoxycarbonylmethyl, with alk in each case being vinylene, or a salt thereof.

4. 7-Fluoro-1-phenylpyrimido[1,6-a]indole-5-acetic acid ethyl ester or a salt thereof.

5. 7-Fluoro-1-(2-thienyl)pyrimido[1,6-a]indole-S-acetic acid ethyl ester or a salt thereof.

6. 1-(2-Thienyl)pyrimido[1,6-a]indole-5-acetic acid ethyl ester or a salt thereof.

7. 7-Methoxy-1-(2-picolinyl)pyrimido[1,6-a]indole-5-acetic acid ethyl ester or a salt thereof.

8. 1-Phenylpyrimido[1,6-a]indole-5-acetic acid or a salt thereof.

9. 7-Fluoro-1-(p-methanesulfinylphenyl)pyrimido[1,6-a]in-dole-5-acetic acid ethyl ester or a salt thereof.

10. 7-Fluoro-1-(p-methanesulfinylphenyl)pyrimido[1,6-a]in-dole-5-acetic acid or a salt thereof.

11. 7-Fluoro-1-phenylpyrimido[1,6-a]indole-5-acetic acid or a salt thereof.

12. 1-(2-Thienyl)pyrimido[1,6-a]indole-5-acetic acid or a salt thereof.

13. 7-Methoxy-1-(2-picolinyl)pyrimido[1,6-a]indole-5-acetic acid or a salt thereof.

14. 7-Fluoro-1-(p-methoxyphenyl)pyrimido[1,6-a]indole-5-acetic acid or a salt thereof.

15. 7-Fluoro-1-(p-methoxyphenyl)pyrimido[1,6-a]indole-5-acetic acid ethyl ester or a salt thereof.

16 7-Methoxy-1-(p-fluorophenyl)pyrimido[1,6-a]indole-5-acetic acid or a salt thereof.

17. 7-Methoxy-1-(p-fluorophenyl)pyrimido[1,6-a]indole-5-acetic acid ethyl ester or a salt thereof.

18. 7-Fluoro-1-(2-thienyl)pyrimido[1,6-a]indole-5-acetic acidor a salt thereof.

19. A compound of formula I according to any one of claims 1 to 18 having antiphlogistic and/or analgesic activity.

20. A pharmaceutical composition containing at least one compound according to any one of claims 1 to 19 in the free form or in the form of a pharmaceutically acceptable salt, together with the customary pharmaceutical adjuvants and carriers.

21. Use of a compound of formula I according to any one of claims 1 to 19 as a medicament.

22. Use of a compound of formula I according to any one of claims 1 to 19 in the treatment of painful conditions or inflammatory processes.

23. Use of a compound of formula I according to any one of claims 1 to 19 for the preparation of a pharmaceutical composition.

24. Use of a compound of formula I according to any one of claims 1 to 19 in a therapeutic method of treating the human or animal body.

25. A process for the preparation of a compound of the general formula I according to any one of claims 1 to 19, or of a salt thereof, which process comprises removing H-$Z_1$ from a compound of the general formula

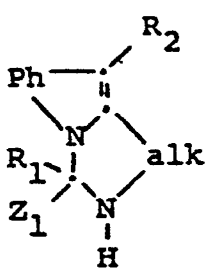

(II)

wherein $Z_1$ is unmodified or functionally modified hydroxy or the mercapto group, or from a salt thereof to introduce an additional bond, or isomerising a compound of the formula

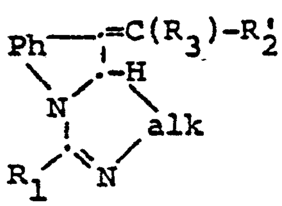

(V)

wherein $R'_2$ is carboxy which is free or esterified as indicated under $R_2$, and $R_3$ is hydrogen, or a salt thereof, or cyclising a compound of the general formula

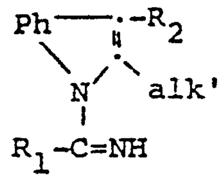

(VIII)

wherein alk' is a group of the formula -CH=CH-$Z_6$ or -CH -CH=$Z'_6$ in which $Z_6$ is unmodified or functionally modified hydroxy or amino and $Z'_6$ is oxo or imino, or a salt thereof, or in a compound of the formula

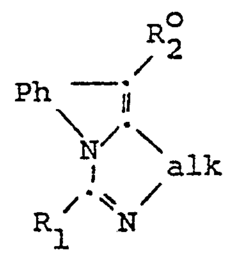

(X)

wherein $R^o_2$ is a radical that can be converted into the group $R_2$, or in a salt thereof, converting $R^o_2$ into the group $R_2$ by solvolysis or oxidation, or dehydrogenating a compound of the formula

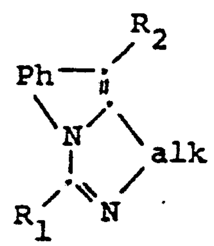

(I)

wherein alk is ethylene, to form an additional bond and if desired, converting a compound obtainable according to the invention into a different compound of formula I or converting a free compound of formula I obtainable according to the invention into a salt or converting a salt obtainable according to the process into the free compound of formula I or into a different salt.

26. A process according to claim 25, which comprises, in a compound of formula X, wherein $R^o_2$ is a group of the formula -CH($R_3$)-$R''_2$, in which $R_3$ is hydrogen and R"2 is functionally modified carboxy other than $R'_2$ and $R'_2$ is identical with the carboxy or lower alkoxycarbonyl moiety of $R_2$, converting $R^o_2$ into $R_2$ by hydrolysis or alcoholysis, or dehydrogenating a compound of formula I, wherein alk is ethylene in the presence of a dehydrogenating agent, to form an additional double bond.

27. A process according to either of claims 25 or 26, which comprises dehydrogenating a compound of the formula

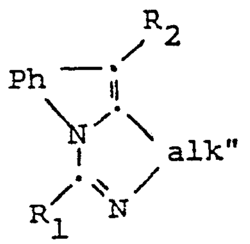

(I)

wherein alk is ethylene, to form an additional bond, and, if desired, in a compound of formula I obtainable according to the invention, in which formula Ph, alk and $R_1$ are as defined above and $R_2$ is lower alkoxycarbonylmethyl, hydrolysing lower alkoxycarbonylmethyl $R_2$ to give carboxymethyl $R_2$.

28. A process for the preparation of a pharmaceutical composition, which comprises combining a compound according to any one of claims 1 to 19 in the free form or in the form of a pharmaceutically acceptable salt with customary pharmaceutical adjuvants and/or carriers.

**Claims** for the Contracting State: AT

1. A process for the preparation of a compound of the general formula

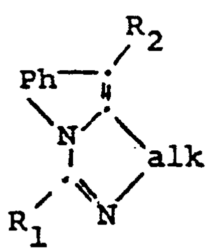

(I)

wherein $R_1$ is phenyl which is unsubstituted or substituted in the p-position by lower alkylthio containing up to and including 4 carbon atoms, by lower alkanesulfinyl containing up to and including 4 carbon atoms, by lower alkoxy containing up to and including 4 carbon atoms or by halogen having an atomic number of up to and including 35, or is thienyl, or pyridyl which is unsubstituted or substituted by lower alkyl containing up to and including 4 carbon atoms, $R_2$ is carboxymethyl or lower alkoxycarbonylmethyl containing up to and including 5 carbon atoms, Ph is 1,2-phenylene which is unsubstituted or substituted in the p-position to the nitrogen atom by lower alkoxy containing up to and including 4 carbon atoms or by halogen having an atomic number of up to and including 35, and alk is vinylene, or of a salt thereof, with the proviso that if $R_1$ is p-methylthiophenyl and Ph is 1,2-phenylene substituted in the p-position to the nitrogen atom by fluorine, then $R_2$ is other than carboxymethyl or ethoxycarbonylmethyl, and with the further proviso that if $R_1$ is phenyl and Ph is unsubstituted 1,2-phenylene or if $R_1$ is p-chlorophenyl and Ph is 1,2-phenylene substituted in the p-position to the nitrogen atom by methoxy, then $R_2$ is other than ethoxycarbonylmethyl, which process comprises removing $H_{-1}$ from a compound of the general formula

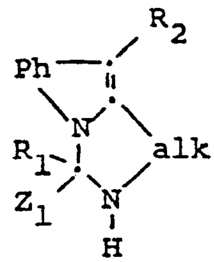

(II)

wherein $Z_1$ is unmodified or functionally modified hydroxy or the mercapto group, or from a salt thereof, to introduce an additional bond, or isomerising a compound of the formula

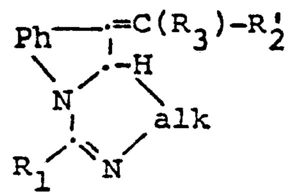

(V)

wherein $R'_2$ is carboxy which is free or esterified as indicated under $R_2$, and $R_3$ is hydrogen, or a salt thereof, or cyclising a compound of the general formula

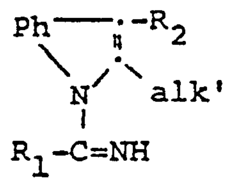

(VIII)

wherein alk' is a group of the formula -CH=CH-$Z_6$ or -CH-CH=$Z'_6$ in which $Z_6$ is unmodified or functionally modified hydroxy or amino and $Z'_6$ is oxo or imino, or a salt thereof, or in a compound of the formula

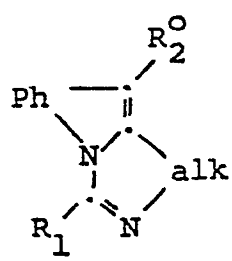

(X)

wherein $R^o_2$ is a radical that can be converted into the group $R_2$, or in a salt thereof, converting $R^o_2$ into the group $R_2$ by solvolysis or oxidation, or dehydrogenating a compound of the formula.

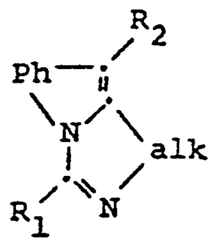

(I)

wherein alk is ethylene, to form an additional bond, and, if desired converting a compound obtainable according to the invention into a different compound of formula I or converting a free compound of formula I obtainable according to the invention into a salt or converting a salt obtainable according to the process into the free compound of formula I or into a different salt.

2. A process according to claim 1, which comprises, in a compound of formula X, wherein R 2 is a group of the formula -CH($R_3$)-$R''_2$, in which $R_3$ is hydrogen and $R''_2$ is functionally modified carboxy other than $R'_2$ and $R'_2$ is identical with the carboxy or lower alkoxycarbonyl moiety of $R_2$, converting $R^o_2$ into $R_2$ by hydrolysis or alcoholysis, or dehydrogenating a compound of formula I, wherein alk is ethylene, in the presence of a dehydrogenating agent, to form an additional double bond.

3. A process according to either of claims 1 or 2 which comprises dehydrogenating a compound of the formula

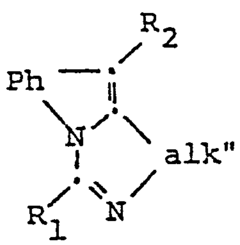

(I)

wherein alk is ethylene, to form an additional bond, and, if desired, in a compound of formula I obtainable according to the invention, in which formula Ph, alk and $R_1$ are as defined above and $R_2$ is lower alkoxycarbonylmethyl hydrolysing lower alkoxycarbonylmethyl $R_2$ to give carboxymethyl $R_2$.

4. A process according to any one of claims 1 to 3 for the preparation of a compound of formula I, wherein $R_1$ is unsubstituted phenyl p-methoxyphenyl or p-methanesulfinylphenyl, $R_2$ is ethoxycarbonyl6ethyl, Ph is 1,2-phenylene substituted in the p-position to the nitrogen atom by fluorine and alk is vinylene, or wherein $R_1$ is unsubstituted 2-thienyl, unsubstituted phenyl, p-methanesulfinylphenyl or p-methoxyphenyl, $R_2$ is carboxymethyl, Ph is 1,2-phenylene substituted in the p-position to the nitrogen atom by fluorine and alk is vinylene, or wherein $R_1$ is unsubstituted 2-thienyl, $R_2$ is carboxymethyl or ethoxycarbonylmethyl, Ph is unsubstituted 1,2-phenylene and alk is vinylene, or wherein $R_1$ is unsubstituted phenyl, $R_2$ is carboxymethyl, Ph is unsubstituted 1,2-phenylene and alk is vinylene, or wherein $R_1$ is 2-picolinyl or p-fluorophenyl, $R_2$ is carboxymethyl or ethoxycarbonylmethyl, Ph is 1,2-phenylene substituted in the p-position to the nitrogen atom by methoxy and alk is vinylene, or of a salt thereof.

5. A process according to any one of claims 1 to 3 for the preparation of a compound of formula I wherein Ph is unsubstituted 1,2-phenylene and $R_1$ is phenyl and $R_2$ is carboxymethyl or $R_1$ is 2-thienyl and $R_2$ is carboxymethyl or ethoxycarbonylmethyl, or Ph is 1,2-phenylene substituted in the p-position to the nitrogen atom by fluorine and $R_1$ is phenyl, p-methanesulfinylphenyl or p-methoxyphenyl and $R_2$ is carboxymethyl or ethoxycarbonylmethyl, or Ph is 1,2-phenylene substituted in the p-position to the nitrogen atom by methoxy and $R_1$ is p-fluorophenyl or 2-picolinyl and $R_2$ is carboxymethyl or ethoxycarbonylmethyl, with alk in each case being vinylene or of a salt thereof.

6. A process according to any one of claims 1 to 3 for the preparation of 7-fluoro-1-(2-thienyl)pyrimido[1,6-a]indole-5-acetic acid or of a salt thereof.

7. A process according to any one of claims 1 to 3 for the preparation of 7-fluoro-1-phenylpyrimido[1,6-a]indole-5-acetic acid ethyl ester or of a salt thereof.

8. A process according to any one of claims 1 to 3 for the preparation of 7-fluoro-1-(2-thienyl)pyrimido[1,6-a]indole-5-acetic acid ethyl ester or of a salt thereof.

9. A process according to any one of claims 1 to 3 for the preparation of 1-(2-thienyl)pyrimido[1,6-a]indole-5-acetic acid ethyl ester or of a salt thereof.

10. A process according to any one of claims 1 to 3 for the preparation of 7-methoxy-1-(2-picolinyl)pyrimido[1,6-a]indole-5-acetic acid ethyl ester or of a salt thereof.

11. A process according to any one of claims 1 to 3 for the preparation of 1-phenylpyrimido[1,6-a]indole-5-acetic acid or of a salt thereof.

12. A process according to any one of claims 1 to 3 for the preparation of 7-fluoro-1-(p-methanesulfinylphenyl)pyrimido-[1,6-a]indole-5-acetic acid ethyl ester or of a salt thereof.

13. A process according to any one of claims 1 to 3 for the preparation of 7-fluoro-1-(p-methanesulfinylphenyl)pyrimido-[1,6-a]indole-5-acetic acid or of a salt thereof.

14. A process according to any one of claims 1 to 3 for the preparation of 7-fluoro-1-phenylpyrimido[1,6-a]indole-5-acetic acid or of a salt thereof.

15. A process according to any one of claims 1 to 3 for the preparation of 1-(2-thienyl)pyrimido[1,6-a]indole-5-acetic acid or of a salt thereof.

16. A process according to any one of claims 1 to 3 for the preparation of 7-methoxy-1-(2-picolinyl)pyrimido[1,6-a]indole-5-acetic acid or of a salt thereof.

17. A process according to any one of claims 1

to 3 for the preparation of 7-fluoro-1-(p-methoxyphenyl)pyrimido[1,6-a]indole-5-acetic acid or of a salt thereof.

18. A process according to any one of claims 1 to 3 for the preparation of 7-fluoro-1-(p-methoxyphenyl)pyrimido[1,6-a]indole-5-acetic acid or of a salt thereof.

19. A process according to any one of claims 1 to 3 for the preparation of 7-methoxy-1-(p-fluorophenyl)pyrimido[1,6-a]indole-5-acetic acid or of a salt thereof.

20. A process according to any one of claims 1 to 3 for the preparation of 7-methoxy-1-(p-fluorophenyl)pyrimido[1,6-a]indole-5-acetic acid ethyl ester or of a salt thereof.

21. A process for the preparation of a pharmaceutical composition, which comprises combining a compound according to any one of claims 1 and 4 to 20 in the free form or in the form of a pharmaceutically acceptable salt with customary pharmaceutical adjuvants and/or carriers.

**Revendications** pour les Etats contractants: DE, FR, CH, LI, IT,NL, BE, SE, LU

1. Amidines d'acides carboxyliques cyclisées sur N,N', de formule générale

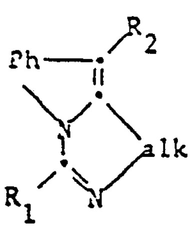

(I)

dans laquelle $R_1$ représente un groupe phényle éventuellement substitué en position para par un groupe alkylthio inférieur contenant jusqu'à 4 atomes de carbone inclus, par un groupe alcane-sulfinyle inférieur contenant jusqu'à 4 atomes de carbone inclus, un groupe alcoxy inférieur contenant jusqu'à 4 atomes de carbone inclus ou un halogène de numéro atomique allant jusqu'à 35 inclus, un groupe thiényle ou un groupe pyridyle éventuellement substitué par un groupe alkyle inférieur contenant jusqu'à 4 atomes de carbone inclus, $R_2$ représente un groupe carboxyméthyle ou (alcoxy inférieur)carbonylméthyle contenant jusqu'à 5 atomes de carbone inclus, Ph représente un groupe 1,2-phénylène éventuellement substitué en position para de l'atome d'azote par un groupe alcoxy inférieur contenant jusqu'à 4 atomes de carbone inclus ou un halogène de numéro atomique allant jusqu'à 35 inclus et alk représente un groupe vinylène, et leurs sels, avec la restriction que, lorsque $R_1$ représente un groupe pméthylthiophényle et Ph un groupe 1,2-phénylène substitué par le fluor en position para de l'atome d'azote, $R_2$ ne peut représenter un groupe carboxyméthyle ou éthoxycarbonylméthyle, et également avec la restriction que, lorsque $R_1$ représente un groupe phényle et Ph un groupe 1,2-phénylène non substitué ou bien lorsque $R_1$ représente un groupe p-chlorophényle et Ph un groupe 1,2-phénylène substitué par un groupe méthoxy en position para de l'atome d'azote, $R_2$, dans chaque cas, ne peut représenter un groupe éthoxycarbonylméthyle.

2. Composés de formule I selon la revendication 1, dans laquelle $R_1$ représente un groupe phényle non substitué, p-méthoxyphényle ou p-méthane-sulfinylphényle, $R_2$ représente dans chaque cas un groupe éthoxycarbonyleméthyle, Ph représente dans chaque cas un groupe 1-2-phénylène substitué par le fluor en position para de l'atome d'azote et alk représente dans chaque cas un groupe vinylène, dans laquelle $R_1$ représente un groupe 2-thiényle non substitué, phényle non substitué, p-méthane-sulfinylphényle ou p-méthoxyphényle, $R_2$ représente dans chaque cas un groupe carboxyméthyle, Ph représente dans chaque cas un groupe 1,2-phénylène substitué par le fluor en position para de l'atome d'azote et alk représente dans chaque cas un groupe vinylène, dans laquelle $R_1$ représente dans chaque cas un groupe 2-thiényle non substitué, $R_2$ représente un groupe carboxyméthyle ou éthoxycarbonylméthyle, Ph représente dans chaque cas un groupe 1,2-phénylène non substitué et alk représente dans chaque cas un groupe vinylène, dans laquelle $R_1$ représente un groupe phényle non substitué, $R_2$ un groupe carboxyméthyle, Ph un groupe 1,2-phénylène non substitué et alk un groupe vinylène, ou bien dans laquelle $R_1$ représente un groupe 2-picolinyle ou p-fluorophenyle, $R_2$ représente dans chaque cas un groupe carboxyméthyle ou éthoxycarbonylméthyle, Ph représente dans chaque cas un groupe 1,2-phénylène substitué par un groupe méthoxy en position para de l'atome d'azote et alk représente dans chaque cas un groupe vinylène, et leurs sels.

3. Composés de formule I selon la revendication 1, dans laquelle Ph représente un groupe 1,2-phénylène non substitué et $R_1$ un groupe phényle et $R_2$ un groupe carboxyméthyle ou bien $R_1$ représente un groupe 2-thiényle et $R_2$ un groupe carboxy- ou éthoxycarbonylméthyle, ou bien Ph représente 1,2-phénylène substitué par le fluor en position para de l'atome d'azote et $R_1$ représente un groupe phényle, p-methane-sulfinylphényle ou p-méthoxyphényle ou bien $R_2$ représente un groupe carboxy- ou éthoxycarbonylméthyle, ou bien Ph représente un groupe 1,2-phénylène substitué par un groupe méthoxy en position para de l'atome d'azote et $R_1$ représente un groupe p-fluorophényle ou 2-picolinyle et $R_2$ un groupe carboxy- ou éthoxycarbonylméthyle, alk représentant dans chaque cas un groupe vinylène, et leurs sels.

4. Le 7-fluoro-1-phényl-pyrimido[1,6-a]indole-5-acétate d'éthyle ou un sel de ce composé.

5. Le 7-fluoro-1-(2-thiényl)-pyrimido[1,6-a]-indole-5-acétate d'éthyle, ou un sel de ce composé.

6. Le 1-(2-thiényl)-pyrimido[1,6-a]indole-5-acétate d'éthyle, ou un sel de ce composé.

7. Le 7-méthoxy-1-(2-picolinyl)-pyrimido[1,6-a]-indole-5-acétate d'éthyle, ou un sel de ce composé.

8. L'acide 1-phényl-pyrimido[1,6-a]indole-5-acétique ou un sel de ce composé.

9. Le 7-fluoro-1-(p-méthane-sulfinylphényl)-pyrimido[1,6-a]indole-5-acétate d'éthyle ou un sel de ce composé.

10. L'acide 7-fluoro-1-(p-méthane-sulfinylphényl)-pyrimido[1,6-a]indole-5-acétique ou un sel de ce composé.

11. L'acide 7-fluoro-1-phényl-pyrimido[1,6-a]-indole-5-acétique ou un sel de ce composé.

12. L'acide 1-(2-thiényl)-pyrimido[1,6-a]indole-5-acétique ou un sel de ce composé.

13. L'acide 7-méthoxy-1-(2-picolinyl)-pyrimido-[1,6-a]indole-5-acétique ou un sel de ce composé.

14. L'acide 7-fluoro-1-(p-méthoxyphényl)-pyrimido-[1,6-a]indole-5-acétique ou un sel de ce composé.

15. Le 7-fluoro-1-(p-méthoxyphényl)-pyrimido-[1,6-a]indole-5-acétate d'éthyle ou un sel de ce composé.

16. L'acide 7-méthoxy-1-(p-fluorophényl)-pyrimido-[1,6-a]indole-5-acétique ou un sel de ce composé.

17. Le 7-méthoxy-1-(p-fluorophényl)-pyrimido-[1,6-a]indole-5-acétate d'éthyle ou un sel de ce composé.

18. L'acide 7-fluoro-1-(2-thiényl)-pyrimido-[1,6-a]indole-5-acétique ou un sel de ce composé.

19. Composé de formule I selon l'une des revendications 1 à 18, possédant une activité antiphlogistique et/ou analgésique.

20. Compositions pharmaceutiques contenant au moins un composé selon l'une des revendications 1 à 19, à l'état libre ou à l'état de sel acceptable pour l'usage pharmaceutique, avec les produits auxiliaires et véhicules pharmaceutiques usuels.

21. Utilisation des composés de formule I selon l'une des revendications 1 à 19, en tant que médicaments.

22. Utilisation des composés de formule I selon l'une des revendications 1 à 19 dans le traitement des états de douleur ou des processus inflammatoires.

23. Utilisation des composés de formule I selon l'une des revendications I à 19 pour la préparation de compositions pharmaceutiques.

24. Composé de formule I selon l'une des revendications 1 à 19 pour l'utilisation dans un procédé pour le traitement thérapeutique de l'organisme humain ou animal.

25. Procédé de préparation des composés de formule générale I selon l'une des revendications 1 à 19, et de leurs sels, caractérisé en ce que, partant de composés de formule générale

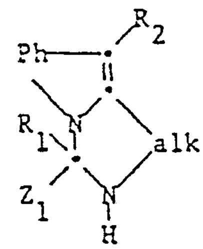

(II)

dans laquelle $Z_1$ représente un groupe hydroxy ayant éventuellement subi une modification fonctionnelle ou le groupe mercapto, ou de sels de tels composés, on elimine $H_{-1}$ en introduisant ainsi une liaison supplémentaire, ou bien on isomérise des composés de formule

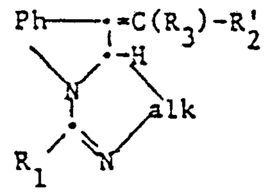

(V)

dans laquelle $R'_2$ represente un groupe carboxy éventuellement estérifié comme indiqué en référence à $R_2$ et $R_3$ représente l'hydrogène, ou des sels de tels composés, ou bien on cyclise un composé de formule générale

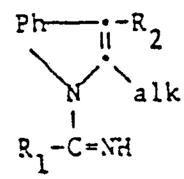

(VIII)

dans laquelle alk' représente un groupe de formule $-CH=CH-Z_6$ ou $-CH_2-CH=Z'_6$, $Z_6$ representant un groupe hydroxy ou amino ayant éventuellement subi une modification fonctionnelle et $Z'_6$ un groupe oxo ou imino, ou un sel d'un tel compose, ou bien, dans un composé de formule

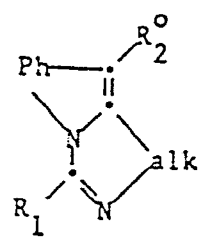

(X)

dans laquelle $R^o_2$ représente un reste convertible en le groupe $R_2$, ou dans des sels d'un tel composé, on convertit le groupe $R_2$, par solvolyse ou oxydation en le groupe $R_2$ ou bien on part de composés de formule

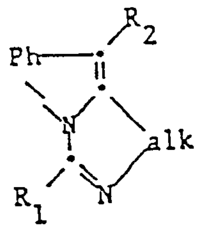

(I)

dans laquelle alk représente un groupe éthylène, qu'on soumet à déshydrogénation avec formation d'une liaison suplémentaire, et si on le désire, on convertit un composé obtenu conformément à l'invention en un autre composé de formule I ou un composé libre obtenu conformément à l'invention et répondant à la formule I en un sel ou un sel obtenu conformément à l'invention en le composé libre de formule I ou en un autre sel.

26. Procédé selon la revendication 25, caractérisé en ce que, dans un composé de formule X, dans laquelle $R^o_2$ représente un groupe de formule $-CH(R_3)-R''_2$ dans laquelle $R_3$ représente l'hydrogène et $R''_2$ est un groupe carboxy ayant subi une modification fonctionnelle et différent de $R'_2$, et $R'_2$ est identique à la partie carboxy ou (alcoxy inférieur)-carbonyle de $R_2$, on convertit $R^o_2$ par hydrolyse ou alcoolyse en $R_2$ ou bien on soumet un composé de formule I dans laquelle alk représente un groupe éthylène à déshydrogénation en présence d'un agent déshydrogénant avec formation simultanée d'une double liaison supplémentaire.

27. Procédé selon la revendication 25 ou 26, caractérisé en ce que l'on soumet des composés de formule

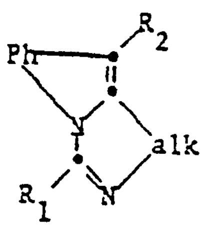

(I)

dans laquelle alk représente un groupe éthylène, à déshydrogénation avec formation simultanée d'une liaison supplémentaire, et si on le désire, dans un composé obtenu supplémentaire, et si on le désire, dans un composé obtenu laquelle Ph, alk et $R_1$ ont les significations indiquées ci-dessus et $R_2$ représente un groupe (alcoxy inférieur)carbonylméthyle, on hydrolyse le groupe (alcoxy inférieur)carbonylméthyle $R_2$ en un groupe carboxyméthyle $R_2$.

28. Procédé de préparation de compositions pharmaceutiques, caractérisé en ce que l'on mélange un composé selon l'une des revendications 1 à 19 à l'état libre ou à l'état de sel acceptable pour l'usage pharmaceutique avec des produits auxiliaires et véhicules pharmaceutiques usuels.

**Revendications** pour l'Etat contractant: AT

1. Procédé de préparation de composés de formule generale

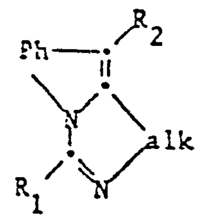

(I)

dans laquelle $R_1$ représente un groupe phényle éventuellement substitué en position para par un groupe alkylthio inférieur contenant jusqu'à 4 atomes de carbone inclus, par un groupe alcane-sulfinyle inférieur contenant jusqu'à 4 atomes de carbone inclus, un groupe alcoxy inférieur contenant jusqu'à 4 atomes de carbone inclus, ou un halogène de numéro atomique allant jusqu'à 35 inclus, un groupe thiényle ou un groupe pyridyle éventuellement substitué par un groupe alkyle inférieur contenant jusqu'à 4 atomes de carbone inclus, $R_2$ représente un groupe carboxyméthyle ou (alcoxy inférieur)-carbonylméthyle contenant jusqu'à 5 atomes de carbone inclus, Ph représente un groupe 1,2-phénylène éventuellement substitué en position para de l'atome d'azote par un groupe alcoxy inférieur contenant jusqu'à 4 atomes de carbone inclus ou un halogène de numéro atomique allant jusqu'à 35 inclus et alk représente un groupe vinylène, et de leurs sels, avec la restriction que lorsque $R_1$ représente un groupe p-méthylthiophényle et Ph un groupe 1,2-phénylène substitué par le fluor en position para de l'atome d'azote, $R_2$ ne peut représenter un groupe carboxymethyle ou éthoxycarbonylméthyle, et également la restriction selon laquelle, lorsque $R_1$ représente un groupe phényle et Ph un groupe 1,2-phénylène non substitué ou bien lorsque $R_1$ représente un groupe p-chlorophényle et Ph un groupe 1,2-phénylène substitué par un groupe méthoxy en position para de l'atome d'azote, $R_2$, dans l'un ou l'autre cas, ne peut représenter un groupe éthoxycarbonyméthyle, ce procédé se caractérisant en ce que, partant de composés de formule générale

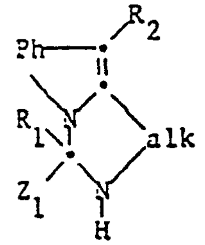

(II)

dans laquelle $Z_1$ représente un groupe hydroxy ayant eventuellement subi une modification fonctionnelle ou le groupe mercapto, ou de sels de tels composés, on élimine $H-Z_1$ en introdusiant une liaison supplémentaire, ou bien on isomérise

des composés de formule

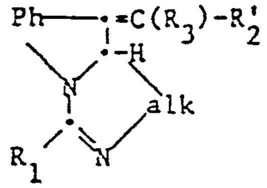

(V)

dans laquelle $R'_2$ représente un groupe carboxy éventuellement estérifié comme indique en reference a $R_2$, et $R_3$ représente l'hydrogene ou des sels de tels composés, ou bien on cyclise un compose de formule générale

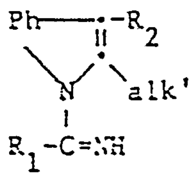

(VIII)

dans laquelle alk' représente un groupe de formule $-CH=CH-Z_6$ ou $-CH_2-CH=Z'_6$, $Z_6$ represente un groupe hydroxy ou amino ayant éventuellement subi une modification fonctionnelle et $Z'_6$ représente un groupe oxo ou imino, ou un sel d'un tel composé, ou bien, dans un composé de formule

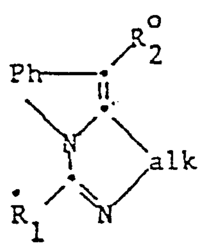

(X)

dans laquelle $R^o_2$ représente un reste convertible en le groupe $R_2$, ou dans des sels d'un tel composé, on convertit $R^o_2$ par solvolyse ou par oxydation en le groupe $R_2$, ou bien on soumet des composés de formule

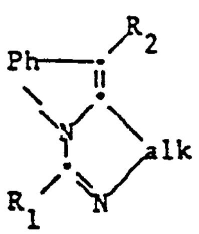

(I)

dans laquelle alk représente un groupe éthylène, à déshydrogénation avec formation simultanée d'une liaison supplémentaire, et si on le désire, on convertit un composé obtenu conformément à l'invention en un autre composé de formule I ou bien on convertit un composé libre obtenu conformément à l'invention et répondant à la formule I en un sel ou un sel obtenu conformément à l'invention en le composé libre de formule I ou en un autre sel.

2. Procédé selon la revendication I, caracterise en ce que dans un composé de formule X dans laquelle $R^o_2$ représente un groupe de formule $-CH(R_3)-R''_2$ dans laquelle $R_3$ représente l'hydrogène et $R''_2$ représente un groupe carboxy ayant subi une modification fonctionnelle et différent de $R'_2$, $R'_2$ étant identique à la partie carboxy ou (alcoxy inférieur)-carbonyle de $R_2$, on convertit $R^o_2$ par hydrolyse ou alcoolyse en $R_2$, ou bien on soumet un composé de formule I dans laquelle alk représente un groupe éthylène à déshydrogénation en présence d'un agent déshydrogénant avec formation simultanée d'une double liaison supplémentaire.

3. Procédé selon les revendications 1 et 2, caractérisé en ce que l'on soumet des composés de formule

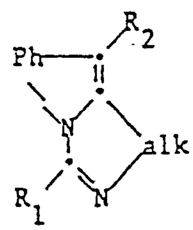

(I)

dans laquelle alk représente un groupe éthylène, à déshydrogénation avec formation simultanée d'une liaison supplémentaire, et si on le désire dans un composé obtenu conformément à l'invention, répondant à la formule I dans laquelle Ph, alk et $R_1$ ont les significations indiquées ci-dessus et $R_2$ représente un groupe (alcoxy inférieur)carbonylméthyle, on hydrolyse le groupe (alcoxy inférieur)carbonylméthyle $R_2$ en un groupe carboxyméthyle.

4. Procédé selon les revendications I à 3 pour la préparation de composés de formule I dans laquelle $R_1$ représente un groupe phényle non substitué, p-méthoxyphényle ou p-méthane-sulfinylphényle, $R_2$ représente dans tous les cas un groupe éthoxycarbonylméthyle, Ph représente dans tous les cas un groupe 1,2-phénylene substitué par le fluor en position para de l'atome d'azote et alk représente dans tous les cas un groupe vinylène, dans laquelle $R_1$ représente un groupe 2-thiényle non substitué, phényle non substitué, p-méthane-sulfinylphényle ou p-méthoxyphényle, $R_2$ représente dans tous les cas un groupe carboxyméthyle, Ph représente dans tous les cas un groupe 1,2-phénylène substitué par le fluor en position para de l'atome d'azote et alk représente dans tous les cas un groupe vinylène, dans laquelle $R_1$ représente dans tous les cas un groupe 2-thiényle non substitué, $R_2$ représente un groupe carboxyméthyle ou éthoxycarbonylméthyle, Ph représente dans tous les cas un groupe 1,2-phénylène non substitué et alk représente dans tous les cas un groupe vinylène, dans laquelle $R_1$ représente un groupe phényle non substitué, $R_2$ un groupe carboxyméthyle, Ph un groupe 1,2-phénylène non substitué et alk un groupe vinylène, ou bien dans laquelle $R_1$ représente un groupe 2-picolinyle ou p-fluorophényle, $R_2$ représente dans les deux cas

un groupe carboxyméthyle ou éthoxycarbonylméthyle, Ph représente dans les deux cas un groupe 1,2-phénylène substitué par un groupe méthoxy en position para de l'atome d'azote et alk représente dans les deux casun groupe vinylène, et leurs sels.

5. Procédé selon les revendications 1 à 3, pour la préparation de composés de formule I dans laquelle Ph représente un groupe 1,2-phénylène non substitué et $R_1$ represente un groupe phényle et $R_2$ un groupe carboxyméthyle ou bien $R_1$ représente un groupe 2-thiényle et $R_2$ un groupe carboxy- ou éthoxycarbonylméthyle, ou bien Ph représente un groupe 1,2-phénylène substitué par le fluor en position para de l'atome d'azote et $R_1$ représente un groupe phényle, p-methane-sulfinylphényle ou p-méthoxyphényle ou bien $R_2$ représente un groupe carboxy- ou éthoxycarbonylméthyle, ou bien Ph représente un groupe 1,2-phénylène substitué par un groupe méthoxy en position para de l'atome d'azote et $R_1$ représente un groupe p-fluorophényle ou 2-picolinyle et $R_2$ represente un groupe carboxy- ou éthoxycarbonylméthyle, alk représentant dans tous les cas un groupe vinylène, et de leurs sels.

6. Procédé selon les revendications 1 à 3 pour la préparation de l'acide 7-fluoro-1-(2-thiényl)-pyrimido-[1,6-a]indole-5-acétique ou d'un sel de cet acide.

7. Procédé selon les revendications 1 à 3, pour la préparation du 7-fluoro-1-phényl-pyrimido[1,6-a]indole-5-acétate d'éthyle ou d'un sel de ce composé.

8. Procédé selon les revendications 1 à 3 pour la préparation du 7-fluoro-1-(2-thiényl)-pyrimido[1,6-a]-indole-5-acétate d'éthyle ou d'un sel de ce composé.

9. Procédé selon les revendications 1 à 3 pour la préparation du 1-(2-thiényl)-pyrimido[1,6-a]indole-5-acétate d'éthyle ou d'un sel de ce compose.

10. Procédé selon les revendications 1 à 3 pour la préparation du 7-méthoxy-1-(2-picolinyl)-pyrimido-[1,6-a]indole-5-acétate d'éthyle ou d'un sel de ce composé.

11. Procédé selon les revendications 1 à 3 pour la préparation de l'acide 1-phényl-pyrimido[1,6-a]indole-5-acétique ou d'un sel de cet acide.

12. Procédé selon les revendications 1 à 3 pour la préparation du 7-fluoro-1-(p-méthane-sulfinylphényl)-pyrimido[16-a]indole-5-acétate d'éthyle ou d'un sel de ce composé.

13. Procédé selon les revendications 1 à 3 pour la préparation de l'acide 7-fluoro-1-(p-méthane-sulfinyl-phényl)-pyrimido[1,6-a]indole-5-acétique ou d'un sel de cet acide.

14. Procédé selon les revendications 1 à 3 pour la préparation de l'acide 7-fluoro-1-phényl-pyrimido[1,6-a]-indole-5-acétique ou d'un sel de cet acide.

15. Procédé selon les revendications 1 à 3 pour la préparation de l'acide 1-(2-thiényl)-pyrimido[1,6-a]-indole-5-acétique ou d'un sel de ce composé.

16. Procédé selon les revendications 1 à 3 pour la préparation de l'acide 7-méthoxy-1-(2-picolinyl)-pyrimido[1,6-a]indole-5-acétique ou d'un sel de cet acide.

17. Procédé selon les revendications 1 à 3 pour la préparation de l'acide 7-fluoro-1-(p-méthoxyphényl)-pyrimido[1,6-a]indole-5-acétique ou d'un sel de cet acide.

18. Procédé selon les revendications 1 à 3 pour la préparation de l'acide 7-fluoro-1-(p-méthoxyphényl)-pyrimido[1,6-a]indole-5-acétique ou d'un sel de cet acide.

19. Procédé selon les revendications 1 à 3 pour la préparation de l'acide 7-méthoxy-1-(p-fluorophényl)-pyrimido[1,6-a]indole-5-acétique ou d'un sel de cet acide.

20. Procédé selon les revendications 1 à 3 pour la préparation du 7-méthoxy-1-(p-fluorophényl)-pyrimido-[1,6-a]indole-5-acétate d'éthyle ou d'un sel de ce compose.

21. Procédé de préparation de compositions pharmaceutiques, caractérisé en ce que'l'on mélange un composé selon l'une des revendications I et 4 à 20 à l'état libre ou à l'état de sel acceptable pour l'usage pharmaceutique, avec des produits auxiliaires et/ou vehicules pharmaceutiques usuels.